(19) Europäisches Patentamt
European Patent Office
Office européen des brevets

(11) EP 0 747 355 A1

(12) EUROPEAN PATENT APPLICATION
published in accordance with Art. 158(3) EPC

(43) Date of publication:
**11.12.1996 Bulletin 1996/50**

(21) Application number: **95907855.1**

(22) Date of filing: **08.02.1995**

(51) Int. Cl.[6]: **C07D 211/46**, C07D 401/06, C07D 405/06, C07D 409/06, C07D 451/04, C07D 453/02, A61K 31/435, A61K 31/44, A61K 31/445

(86) International application number:
**PCT/JP95/00168**

(87) International publication number:
**WO 95/21820 (17.08.1995 Gazette 1995/35)**

(84) Designated Contracting States:
**AT BE CH DE DK ES FR GB GR IE IT LI LU NL PT SE**

(30) Priority: **10.02.1994 JP 16829/94**
**04.03.1994 JP 35064/94**
**17.05.1994 JP 102579/94**
**16.09.1994 JP 221335/94**
**31.10.1994 JP 267412/94**

(71) Applicant: **YAMANOUCHI PHARMACEUTICAL CO. LTD.**
**Tokyo 103 (JP)**

(72) Inventors:
- **TAKEUCHI, Makoto**
  **9-14, Matsumaedai 7-chome**
  **Ibaraki 302-01 (JP)**
- **NAITO Ryo**
  **Ibaraki 305 (JP)**
- **MORIHIRA, Koichiro**
  **22-5-302, Kitasenzoku 1-chome**
  **Tokyo 145 (JP)**
- **HAYAKAWA, Masahiko**
  **Ibaraki 305 (JP)**
- **IKEDA, Ken**
  **Chiba 270-11 (JP)**
- **ISOMURA, Yasuo**
  **Ibaraki 302-01 (JP)**

(74) Representative: **Geering, Keith Edwin**
**REDDIE & GROSE**
**16 Theobalds Road**
**London WC1X 8PL (GB)**

# (54) NOVEL CARBAMATE DERIVATIVE AND MEDICINAL COMPOSITION CONTAINING THE SAME

(57) Carbamate derivatives represented by the general formula (I), salts thereof, hydrates thereof or solvates thereof

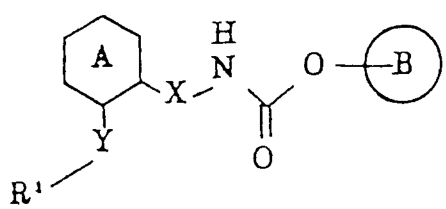

( I )

wherein each symbol has the following meaning:

A ring: a benzene ring or a pyridine ring,
B ring: a nitrogen-containing saturated hetero-ring which may have a substituent on the nitrogen atom and which may have a cross-linking,
$R^1$: a phenyl group which may have a substituent, a cycloalkyl or cycloalkenyl group having 3 to 8 carbon atoms or a five- or six-membered nitrogen-containing heterocyclic group,

EP 0 747 355 A1

X: a single bond or a methylene group,

Y: a single bond, a carbonyl group, a methylene group which may be substituted with a hydroxyl group or a group represented by the formula $-S(O)_{\ell}-$, and

$\ell$: an integer of 0, 1 or 2.

They have muscarinic $M_3$ receptor antagonism and are useful as an agent for the prevention and treatment of gastrointestinal diseases, respiratory diseases or urinary diseases.

## Description

### TECHNICAL FIELD

This invention relates to medicines, particularly, carbamate derivatives having muscarinic receptor antagonizing effects, salts thereof, hydrates thereof or solvates thereof as well as pharmaceutical compositions which contain said compounds.

### BACKGROUND ART

Based on the studies on muscarinic receptors so far made, it is known that a compound having a muscarinic receptor antagonizing effect induces bronchodilation, gastrointestinal motility repression, gastric acid secretion repression, dry mouth, mydriasis, bladder contraction repression, hypohidrosis, tachycardia and the like. It is known that muscarinic receptors exist in at least three subtypes. Mainly, $M_1$ receptors are present in the brain and the like, and $M_2$ receptors in the heart and the like and $M_3$ receptors in smooth muscles and gland tissues.

A number of compounds are known which have affinity for muscarinic receptors. Particularly, atropine (Merck Index, 11th ed., p.138) is used mainly as an antispasmodic agent, because it has high affinity for muscarinic receptors and blocks their functions. However, since atropine shows its affinity for all of the muscarinic receptor subtypes $M_1$, $M_2$ and $M_3$ almost equally and antagonizes non-selectively (*Seitai-no Kagaku* (Medical Science), 42(5), 381 (1991)), it causes side effects such as palpitation, dry mouth, nausea, mydriasis and the like which seem to be induced by muscarinic receptor antagonizing effects other than the desired effect. Of these side effects, particular concern has been directed toward the improvement of heart-related side effects due to the $M_2$ receptor.

In recent years, studies are carried out on compounds which antagonize muscarinic receptors selectively. For example, the unexamined published British patent application No. 2,249,093 discloses N-substituted piperidin-4-ol ester derivatives having the following general formula.

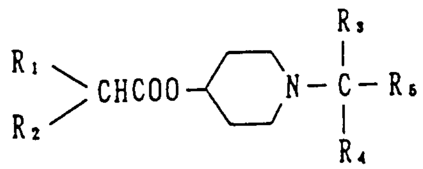

(for the definition of $R_1$ to $R_5$ in the formula, see the above-described published application.)

However, selectivity of these compounds for muscarinic $M_3$ receptor are not sufficient, and these compounds are clearly different from the compounds of the present invention in terms of their structural characteristics that these compounds have ester bonds in their basic structures.

As a patent which discloses carbamate derivatives, an unexamined published Japanese patent application (*Kokai*) No. 4-95071 may be cited. This published patent application discloses compounds having the following general formula.

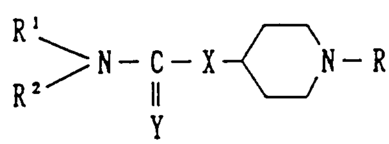

(for the definition of R, $R^1$, $R^2$, X and Y in the formula, see the above-described published application.)

Though this patent generally discloses a phenyl group which may have at least one substituent as an example of the group $R^2$ in the compound represented by the above general formula, Example 13 merely discloses a compound which has a 4-biphenylyl group (phenyl group having a phenyl group at the para-position as a substituent).

On the other hand, compounds of the present invention are clearly different in view of their structures in which, as will be described later as the general formula (I), X and Y which bind to an A ring bind to the carbon atoms adjacent to each other on the A ring (namely, Y is always at the ortho-position to X).

In addition, compounds described above are disclosed as compounds which have antiamnestic effects and the muscarinic receptor-related functions are not disclosed, so that these compounds are clearly different from the compounds of the present invention also in terms of pharmacological effects.

In addition, an unexamined published Japanese patent application (*Kokai*) No. 62-209077 discloses compounds represented by the following general formula.

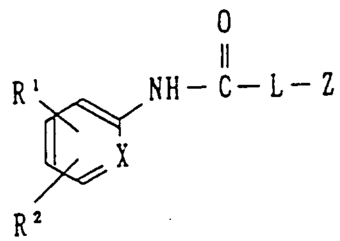

(for the definition of L, X, $R^1$, $R^2$ and Z in the formula, see the above-described published application.)

That is, in the broad definition of the general formula, this patent discloses

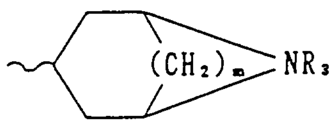

and

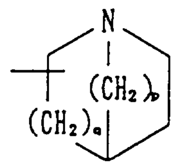

(see the above-described published application for the definition of symbols in the formula) as Z and a moiety which can bind to NH group by hydrogen bonding, illustratively C-$OR^5$ (see the above patent for the definition of the symbol in the formula), as X.

As illustrative compounds, it discloses an urea derivative in which Z is 1-azabicyclo[2.2.2]oct-3-yl group and an aromatic ring containing X is 2-methoxyphenyl group (Example E14 in the above-described published application) and a carbamate derivative in which Z is 8-methyl-8-azabicyclo[3.2.1]oct-3-yl group and an aromatic ring containing X is 2-phenoxyphenyl group (Example E27 in the above-described published application).

However, compounds of the present invention are clearly different in view of their structures in which Y in the following general formula are not oxygen atom and 2-methoxyphenyl group or 2-phenoxyphenyl group is not possible as A ring-Y-$R^1$. Also, compounds described above are disclosed as compounds which have 5-HT antagonistic activity and their muscarinic receptor antagonizing effects are not disclosed, so that these compounds are clearly different from the compounds of the present invention also in view of pharmacological effects.

DISCLOSURE OF THE INVENTION

The inventors of the present invention have carried out extensive studies on compounds capable of showing selective antagonizing effects for the muscarinic $M_3$ receptor and succeeded in creating novel carbamate derivatives having the following general formula (I) which are possessed of excellent selective antagonizing effects for the muscarinic $M_3$ receptor, and have accomplished the present invention on the basis of this finding.

Accordingly, the present invention relates to carbamate derivatives represented by the following general formula (I), salts thereof, hydrates thereof or solvates thereof, as well as a muscarinic $M_3$ receptor antagonizing agents comprising said compounds or pharmaceutically acceptable salts thereof.

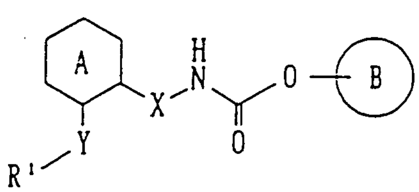

( I )

wherein each symbol has the following meaning:

A ring: a benzene ring or a pyridine ring,

B ring: a nitrogen-containing saturated hetero-ring which may have a substituent on the nitrogen atom and which may have a cross-linking,

$R^1$: a phenyl group which may have a substituent, a cycloalkyl or cycloalkenyl group having 3 to 8 carbon atoms or a five- or six-membered nitrogen-containing heterocyclic group,

X: a single bond or a methylene group,

Y: a single bond, a carbonyl group, a methylene group which may be substituted with a hydroxyl group or a group represented by the formula $-S(O)_{\ell}-$, and

$\ell$: an integer of 0, 1 or 2.

Preferred examples of compounds of the present invention are carbamate derivatives or salts thereof in which the B ring in the above-described general formula (I) are a group represented by any one of the following general formulae (IIa), (IIb) and (IIc)

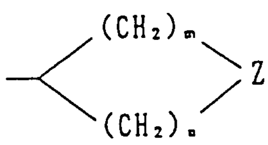
(II a)

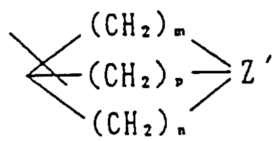
(II b)

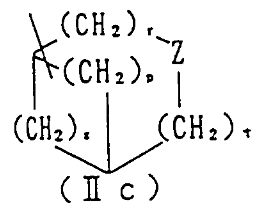
(II c)

wherein each symbol has the following meaning: the same applies hereinafter.

Z: a group represented by

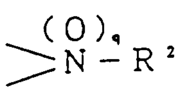
or
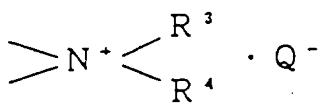
,

Z': a group represented by $\geqslant N(O)_q$ or $\geqslant N^+\text{-}R^5 \cdot Q^-$,

$Q^-$: an anion,

$R^2$: a hydrogen atom, a lower alkyl group, a lower alkenyl group, a lower alkynyl group, a cycloalkyl-lower alkyl group, an aralkyl group which may have a substituent, or a lower alkyl group substituted with a heterocyclic group which has 1 or 2 hetero atoms, which may have a substituent and which may be condensed,

$R^3$: a lower alkyl group, a lower alkenyl group, a lower alkynyl group, an aralkyl group which may have a substituent or a lower alkyl group substituted with a heterocyclic group which has 1 or 2 hetero atoms, which may have a substituent and which may be condensed,

$R^4$: a lower alkyl group, a lower alkenyl group or a lower alkynyl group,

$R^5$: a lower alkyl group, a lower alkenyl group, a lower alkynyl group or an aralkyl group,

m and n: these may be the same or different from each other and each is an integer of from 1 to 4 (with the proviso that m + n means an integer of from 3 to 5),

p: an integer of from 1 to 3 (with the proviso that m + p means an integer of from 3 to 5),

q: 0 or 1, and

r, s and t: these may be the same or different from one another and each is an integer of from 0 to 3 (with the proviso that r + s + t means 2 or 3).

More preferred ones include carbamate derivatives or salts thereof in which the B ring is a group represented by the general formula (IIb),

carbamate derivatives or salts thereof in which the B ring is a group represented by the general formula (IIc),

carbamate derivatives or salts thereof in which $R^1$ is a phenyl group which may have a substituent or a five-membered nitrogen-containing heterocyclic group, or

carbamate derivatives or salts thereof in which the B ring is a group represented by the general formula (IIa), wherein Z is a group represented by the formula

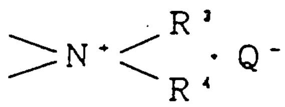 .

In addition, the muscarinic $M_3$ receptor antagonists which contains carbamate derivatives of the present invention represented by the aforementioned general formula (I) or salts thereof as active ingredients are muscarinic $M_3$ receptor antagonists as drugs for use in the prevention or treatment of various diseases, particularly in which the muscarinic $M_3$ receptors are concerned, including respiratory diseases such as chronic obstructive pulmonary disease, chronic bronchitis, asthma, rhinitis and the like, urinary diseases such as urinary incontinence, pollakiuria and the like in neurogenic pollakiuria, neurogenic bladder, nocturnal enuresis, unstable bladder, cystospasm, chronic cystitis and the like and gastrointestinal diseases such as irritable bowel syndrome, spastic colitis, diverticulitis and the like.

The following describes the compound of the present invention further in detail.

Examples of the group represented by the A ring include benzene ring and pyridine ring, and X and Y bind carbon atoms adjacent to each other on the A ring. That is, Y always binds at the ortho-position for X.

The "nitrogen-containing saturated ring which may have a substituent on the nitrogen atom and may have a cross-linking" represented by the B ring is a saturated ring which contains a nitrogen atom in its ring structure as represented by the aforementioned general formula (IIa), (IIb) or (IIc). In the B ring of the general formula (IIa) or (IIb), m + n or m + p is an integer of from 3 to 5, so that it is a five- to seven-membered ring. The B ring of the general formula (IIc) will be described later.

The following illustrates a compound ($Ia^1$) in which Z is represented by

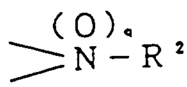

and a compound ($Ia^2$) in which Z is represented by $>N^+(R^3)R^4 \cdot Q^-$, with respect to the cases in which the B ring is represented by the general formula (IIa). Illustrative examples of the B ring represented by the general formula (IIa) include five- to seven-membered rings such as a pyrrolidine ring, a piperidine ring and a hexahydroazepine ring.

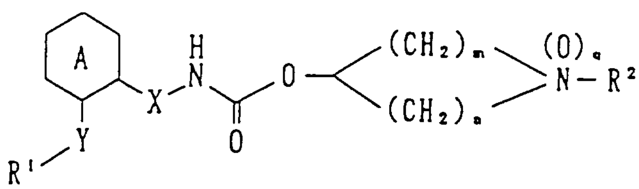 ($Ia^1$)

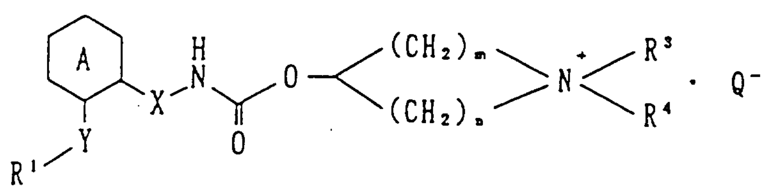 ($Ia^2$)

When the B ring is represented by the general formula (IIb) or (IIc), a cross-linking is present in the B ring in each case. In the general formula (IIb), one nitrogen atom and one carbon atom are bridge heads, while two carbon atoms are bridge heads in the general formula (IIc). When the B ring is represented by the general formula (IIb) or (IIc), the position of the carbon atom which links with the oxygen atom may be any carbon atom on the ring.

The following illustrates a compound ($Ib^1$) in which Z' is represented by $\geqslant N(O)_q$ and a compound ($Ib^2$) in which Z' is represented by $\geqslant N^+-R^5 \cdot Q^-$, with respect to the cases in which the B ring is represented by the general formula (IIb). Illustrative examples of the B ring represented by general formula (IIb) include five- to seven-membered rings such as a quinuclidinyl group, a 1-azabicyclo[2.2.1]heptyl group, a 1-azabicyclo[3.2.1]octyl group and the like, and a bridge head carbon atom or a carbon atom adjacent thereto is desirable as the binding position with the oxygen atom.

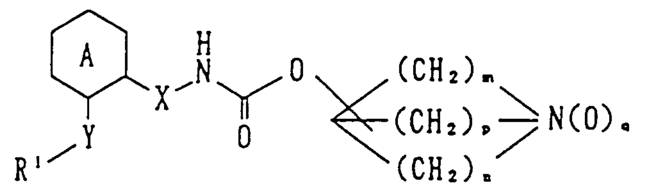

(Ib¹)

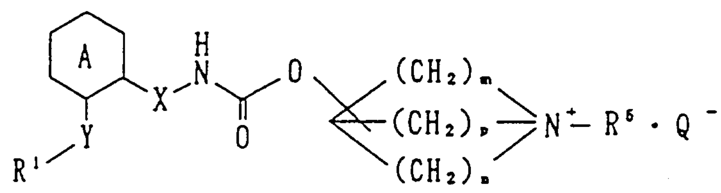

(Ib²)

The following illustrates a compound ($Ic^1$) in which Z is represented by

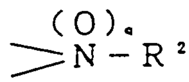

and a compound ($Ic^2$) in which Z is represented by $>N^+(R^3)R^4 \cdot Q^-$ with respect to the cases in which the B ring is represented by the general formula (IIc)

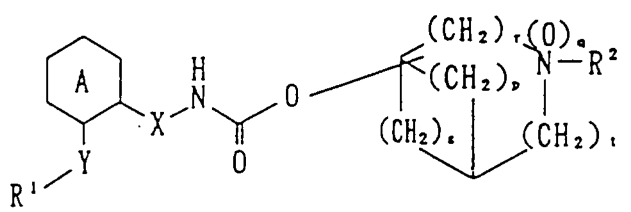

(Ic¹)

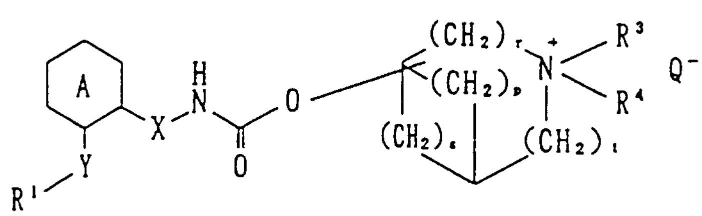

(Ic²)

The following illustrates the B ring represented by the general formula (IIc) in which the total of r, s and t is 3. The cases in which p is 1 are shown in the left side column, the cases in which p is 2 are shown in the central column and the cases in which p is 3 are shown in the right side column. The cases in which the total of r, s and t is 2 can also be illustrated in the same manner.

| | p | | |
|---|---|---|---|
| rst | 1 | 2 | 3 |
| 300 | 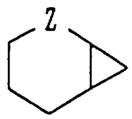 | 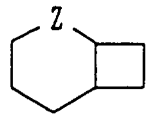 | 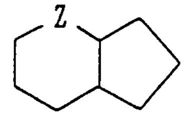 |
| 030 | 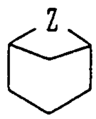 | 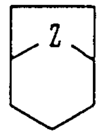 | 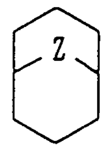 |
| 210 | 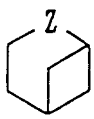 | 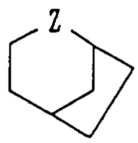 | 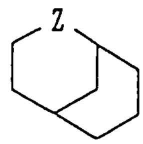 |
| 120 | 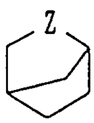 | 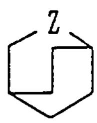 | 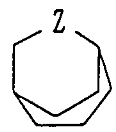 |
| 111 | 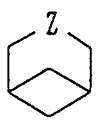 | 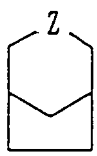 | 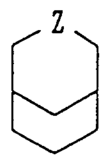 |
| 201 | 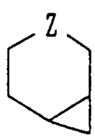 | 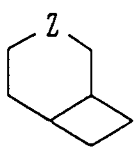 | 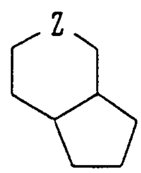 |

The following groups are preferred as the B ring represented by the general formula (IIc).

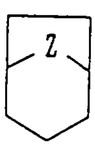 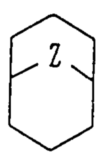 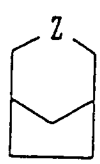 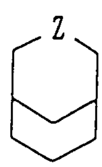 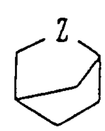

Though not particularly limited, illustrative examples of the anion of the quaternary ammonium salt represented by "$Q^-$" include a halogen atom ion, trifluoromethanesulfonate, p-toluenesulfonate, methanesulfonate and the like, and the halogen atom ion, namely a halide ion (such as a chloride ion, a bromide ion, an iodide ion or the like), is particularly preferred. Illustrative examples of other anions include inorganic anions such as nitrate ion, sulfate ion, phosphate ion, carbonate ion and the like, carboxylate ions such as of formate ($HCOO^-$), acetate ($CH_3COO^-$), propionate, oxalate, malonate and the like and amino acid anions such as of glutamic acid and the like. In the halide ions described above, a bromide ion or an iodide ion is preferred. In this connection, an anion can be converted into another preferred anion

by the usual ion exchange reaction.

The term "lower alkyl group" as used herein means straight or branched alkyl groups each having 1 to 6 carbon atoms. Illustrative examples of the lower alkyl group include methyl, ethyl, propyl, isopropyl, butyl, isobutyl, sec-butyl, tert-butyl, pentyl, isopentyl, neopentyl, tert-pentyl, 1-methylbutyl, 2-methylbutyl, 1,2-dimethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1,3-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, 1-ethyl-2-methylpropyl and the like. Of these groups, alkyl groups having 1 to 4 carbon atoms such as methyl, ethyl, propyl, isopropyl, butyl and the like are preferred, methyl and ethyl are more preferable, and methyl is the most preferable.

The "lower alkenyl group" means straight or branched alkenyl groups each having 2 to 6 carbon atoms, and illustrative examples thereof include vinyl, propenyl, butenyl, methylpropenyl, ethylpropenyl, dimethylvinyl, pentenyl, methylbutenyl, dimethylpropenyl, ethylpropenyl, hexenyl, dimethylbutenyl, methylpentenyl and the like. Propenyl and butenyl groups are preferred, and the propenyl group is more preferred.

The "lower alkynyl group" means straight or branched alkynyl groups each having 2 to 6 carbon atoms, and illustrative examples thereof include ethynyl, propynyl, butynyl, methylpropynyl, pentynyl, methylbutynyl, hexynyl and the like. Of these groups, ethynyl and propynyl are preferred and ethynyl is more preferred.

The term "cycloalkyl-lower alkyl group" as used herein means a group in which an optional hydrogen of the aforementioned lower alkyl group is substituted by a cycloalkyl group, such as cyclohexylmethyl, cyclohexylethyl, cyclohexylpropyl, cyclopentylmethyl and the like, of which cyclohexylmethyl and cyclopentylmethyl are preferred. Illustrative examples of the "cycloalkyl group" include cycloalkyl groups having 3 to 8 carbon atoms, such as cyclopropyl, cyclobutyl, cyclopentyl, cyclohexyl, cycloheptyl, cyclooctyl and the like. Of these groups, cyclopropyl, cyclobutyl, cyclopentyl and cyclohexyl are preferred and cyclobutyl, cyclopentyl and cyclohexyl are more preferred.

The "aralkyl group" is a group derived from the aforementioned "lower alkyl group" by substituting its optional hydrogen atom with an aryl group, and its illustrative examples include benzyl, phenylethyl, phenylpropyl, 2-phenylpropyl and the like of which benzyl and phenylethyl are preferred. The term "aryl group" as used herein means an aromatic hydrocarbon group, preferably an aryl group having 6 to 14 carbon atoms. Its illustrative examples include phenyl, naphthyl, indenyl, anthryl and phenanthryl, of which phenyl or naphthyl is preferred.

The term "aralkyl group which may have a substituent" as used herein means that the aryl moiety of the just described aralkyl group may be substituted with one or a plurality of substituent(s). Their illustrative examples include a halogen atom, a carboxyl group, a nitro group, a cyano group, a hydroxyl group, a trihalogenomethyl group, a lower alkyl group, a lower alkoxy group, a lower alkoxycarbonyl group, a lower acyl group, a mercapto group, a lower alkylthio group, a sulfonyl group, a lower alkylsulfonyl group, a sulfinyl group, a lower alkylsulfinyl group, a sulfonamide group, a lower alkanesulfonamide group, a carbamoyl group, a thiocarbamoyl group, a mono- or di-lower alkylcarbamoyl group, an amino group, a mono- or di-lower alkylamino group, a pyrrolidinyl group, a lower acylamino group, an amidino group, a methylenedioxy group, an ethylenedioxy group, a phenyl group and the like. With regard to the lower alkyl group, it may be substituted with one or plural hydroxyl group, a lower alkoxy group, an amino group and a mono- or di-lower alkylamino group.

Examples of the "halogen atom" include fluorine, chlorine, bromine and iodine. When two or more halogen atoms are substituted, any combination of these atoms may be used. In addition, when the substituent is a halogen atom, the number of substituents is not particularly limited.

Illustrative examples of the "trihalogenomethyl group" include trifluoromethyl, trichloromethyl, tribromomethyl, triiodomethyl, dichlorobromomethyl and the like. Of these groups, trifluoromethyl is preferred.

Illustrative examples of the "lower alkoxy group" include methoxy, ethoxy, propoxy, isopropoxy, butoxy, isobutoxy, sec-butoxy, tert-butoxy, pentyloxy (amyloxy), isopentyloxy, tert-pentyloxy, neopentyloxy, 2-methylbutoxy, 1,2-dimethylpropoxy, 1-ethylpropoxy, hexyloxy and the like. Of these groups, lower alkoxy groups having an alkyl group of 1 to 4 carbon atoms such as methoxy, ethoxy, propoxy, butoxy and the like are preferred, and methoxy and ethoxy are more preferred.

Illustrative examples of the "lower alkoxycarbonyl group" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxy(amyloxy)carbonyl, isopentyloxycarbonyl, tert-pentyloxycarbonyl, neopentyloxycarbonyl, 2-methylbutoxycarbonyl, 1,2-dimethylpropoxycarbonyl, 1-ethylpropoxycarbonyl, hexyloxycarbonyl and the like.

Illustrative examples of the "lower acyl group" include formyl, acetyl, propionyl, butylyl, valeryl, pivaloyl and the like of which formyl, acetyl and propionyl are preferred.

The term "lower alkylthio group" as used herein means a group in which a hydrogen atom of a mercapto group is substituted with the aforementioned lower alkyl group, and its illustrative examples include methylthio, ethylthio, propylthio, isopropylthio, butylthio, pentylthio, hexylthio and the like.

Illustrative examples of the "lower alkylsulfonyl group" include methylsulfonyl, ethylsulfonyl, propylsulfonyl, isopropylsulfonyl, butylsulfonyl, pentylsulfonyl, hexylsulfonyl and the like.

Illustrative examples of the "lower alkylsulfinyl group" include methylsulfinyl, ethylsulfinyl, propylsulfinyl, isopropylsulfinyl, butylsulfinyl, pentylsulfinyl, hexylsulfinyl and the like.

Illustrative examples of the "lower alkanesulfonamide group" include methanesulfonamide, ethanesulfonamide, propanesulfonamide, isopropanesulfonamide, butanesulfonamide, pentanesulfonamide, hexanesulfonamide and the like.

The term "mono- or di-lower alkylcarbamoyl group" as used herein means a carbamoyl group in which one or two hydrogen atoms of the carbamoyl group are substituted with the aforementioned lower alkyl group, and its illustrative examples include methylcarbamoyl, ethylcarbamoyl, propylcarbamoyl, dimethylcarbamoyl and the like.

The term "mono- or di-lower alkylamino group" as used herein means an amino group in which one or two hydrogen atoms of the amino group are substituted with the aforementioned lower alkyl group, and its illustrative examples include methylamino, ethylamino, propylamino, dimethylamino, diethylamino, dipropylamino and the like.

The term "lower acylamino group" as used herein means an amido group in which one or two hydrogen atoms of the amido group are substituted with the aforementioned lower acyl group, and its illustrative examples include acetamido, propionamido, butanamido, isobutanamido, valeramido, hexaneamido and the like.

The term "lower alkyl group substituted with a heterocyclic group which has 1 or 2 hetero atoms, which may have a substituent and which may be condensed" as used herein means a group in which the aforementioned lower alkyl group is substituted with an unsaturated or saturated monocyclic or condensed heterocyclic group. A preferred example of said heterocyclic group is a monocyclic or bicyclic unsaturated heterocyclic group having one or two hetero atoms selected from an oxygen atom, a sulfur atom and a nitrogen atom. Its illustrative examples include furyl, thienyl, pyrrolyl, imidazolyl, pyrazolyl, isothiazolyl, isoxazolyl, pyridyl, pyrimidinyl, pyridazinyl, pyrazinyl, indolyl, indazolyl, indozinyl, quinolyl, quinazolinyl, quinolizinyl, quinoxalinyl, cinnolinyl, benzimidazolyl, imidazopyridyl, benzofuranyl, dihydrobenzofuranyl, naphthylidinyl, 1,2-benzoisoxazolyl, benzoxazolyl, benzothiazolyl, oxazolopyridyl, isothiazolopyridyl, benzothienyl and the like. Of these groups, furyl, thienyl, pyrrolyl, imidazolyl, pyridyl, pyrazinyl, benzimidazolyl, quinolyl, dihydrobenzofuranyl and the like are preferred.

These heterocyclic groups may have a substituent such as a lower alkyl group, an amino group, a nitro group, a cyano group, a halogen atom, a triphenylmethyl (trityl) group or the like.

Examples of the substituent on the "phenyl group which may have a substituent" include a halogen atom, a nitro group, a cyano group, a trihalogenomethyl group, an amino group, a mono- or di-lower alkylamino group, a hydroxyl group, a mercapto group, a lower alkyl group, a lower alkenyl group, a lower alkynyl group, a lower alkoxy group and the like. Of these groups, nitro, amino, hydroxyl, lower alkyl and lower alkoxy are preferred.

Illustrative examples of the "cycloalkenyl group" include those which have 3 to 8 carbon atoms, such as 1-cyclopropenyl, 2-cyclopropenyl, 1-cyclobutenyl, 2-cyclobutenyl, 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 1-cyclooctenyl, 2-cyclooctenyl, 3-cyclooctenyl, 4-cyclooctenyl, 2,4-cyclopentadienyl, 2,5-cyclohexadienyl, 2,4-cycloheptadienyl, 2,6-cycloheptadienyl and the like of which 2-cyclopentenyl and 2-cyclohexenyl are preferred.

The term "five- or six-membered nitrogen-containing heterocyclic group" as used herein means a group which contains at least one nitrogen atom as a ring-constituting atom other than carbon atoms and may also contain another atom selected from oxygen and sulfur, and its illustrative examples include saturated heterocyclic groups such as piperidinyl, pyrrolidinyl, morpholinyl and the like and aromatic heterocyclic groups such as pyrolyl, pyridyl, thiazolyl, imidazolyl, oxazolyl and the like, of which piperidinyl and pyrolyl are preferred.

In some cases, the compound (I) of the present invention may contain one or plural asymmetric carbon atoms, so that it may exist in optical isomers such as the (R) form, the (S) form and the like, as well as in racemates, diastereomers and the like, due to the asymmetric carbon atoms. Also, it may exist in geometrical isomer forms such as the (Z) form, the (E) form and the like due to the presence of double bond depending on the substituents. Mixtures and separated forms of these isomers are included in the scope of the present invention.

Some members of the compound (I) of the present invention can form salts with acids. Examples of such salts include acid addition salts with mineral acids such as hydrochloric acid, hydrobromic acid, hydroiodic acid, sulfuric acid, nitric acid, phosphoric acid and the like or with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, ethanesulfonic acid, glutamic acid and the like. In addition, the compound (I) of the present invention may be isolated as a hydrate, a solvate with ethanol or the like or a polymorphic form.

(Preparation Method)

The compound (I) of the present invention can be prepared by applying various preparation methods. The following describes its typical preparation methods.

First Preparation Method

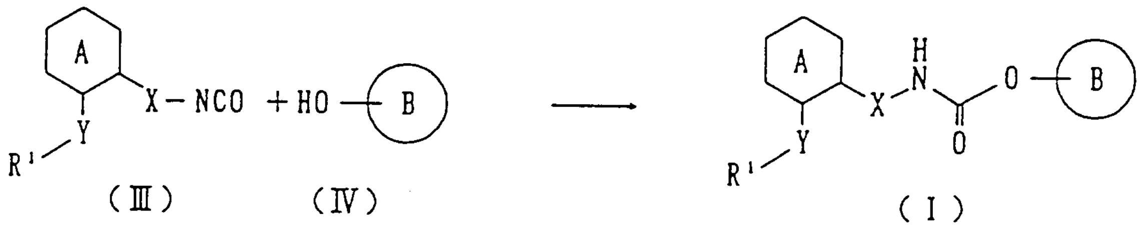

(III) (IV) (I)

(In the above reaction formula, A ring, B ring, $R^1$, X and Y are as defined in the foregoing.)

The compound (I) of the present invention can be obtained by effecting condensation of a isocyanate compound represented by the general formula (III) with an alcohol compound represented by the general formula (IV).

This reaction is carried out by stirring the compound (III) and the compound (IV) in an amount corresponding to the reaction in an inert solvent at room temperature to reflux temperature.

Examples of the inert solvent include dimethylformamide (DMF), dimethylacetamide, tetrachloroethane, dichloromethane, dichloroethane, chloroform, carbon tetrachloride, tetrahydrofuran, dioxane, dimethoxyethane, ethyl acetate, benzene, toluene, acetonitrile, dimethyl sulfoxide and the like, as well as mixed solvents thereof, which may optionally be selected depending on various reaction conditions.

Second Preparation Method

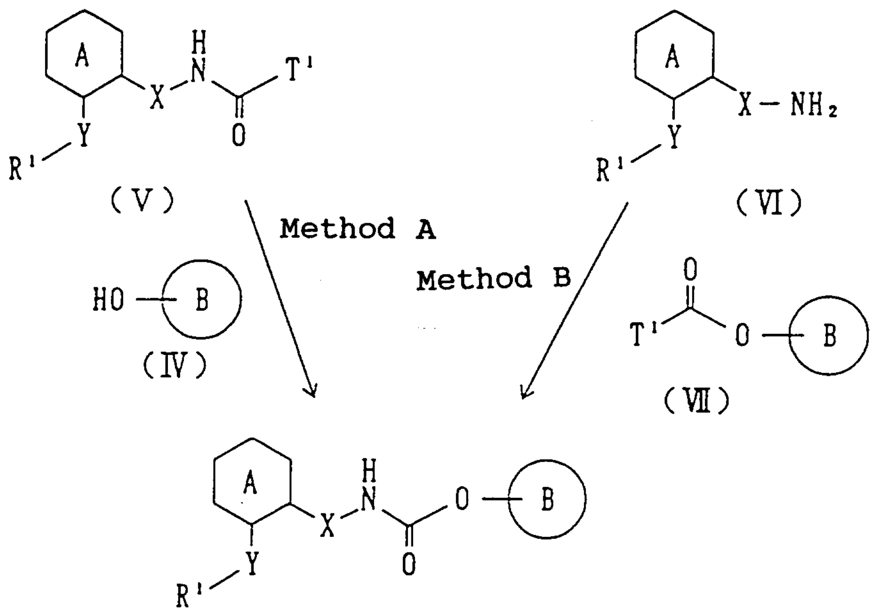


(V) (VI) (IV) (VII)

(I)

(In the above reaction formula, A ring, B ring, $R^1$, X and Y are as defined in the foregoing, and $T^1$ represents a leaving group such as a halogen atom, a lower alkoxy group, a phenoxy group, an imidazolyl group or the like.)

Method A: The compound (I) of the present invention can be obtained by allowing a compound represented by the general formula (V) to react with an alcohol compound represented by the general formula (IV).

This reaction is carried out by stirring the compound (V) and the compound (IV) in an amount corresponding to the reaction in the aforementioned inert solvent at ice-cooled temperature to room temperature or with heating as occasion demands.

In order to accelerate the reaction, it is desirable to add a Lewis acid (e.g., aluminum triisopropoxide or the like) or

a base (e.g., sodium, sodium hydride, sodium methoxide, sodium ethoxide, sodium hydroxide, potassium hydroxide or the like).

Method B: The compound (I) of the present invention can be obtained by allowing a compound represented by the general formula (VI) to react with a compound represented by the general formula (VII). This reaction is carried out by the same treatment of the Method A.

Third Preparation Method

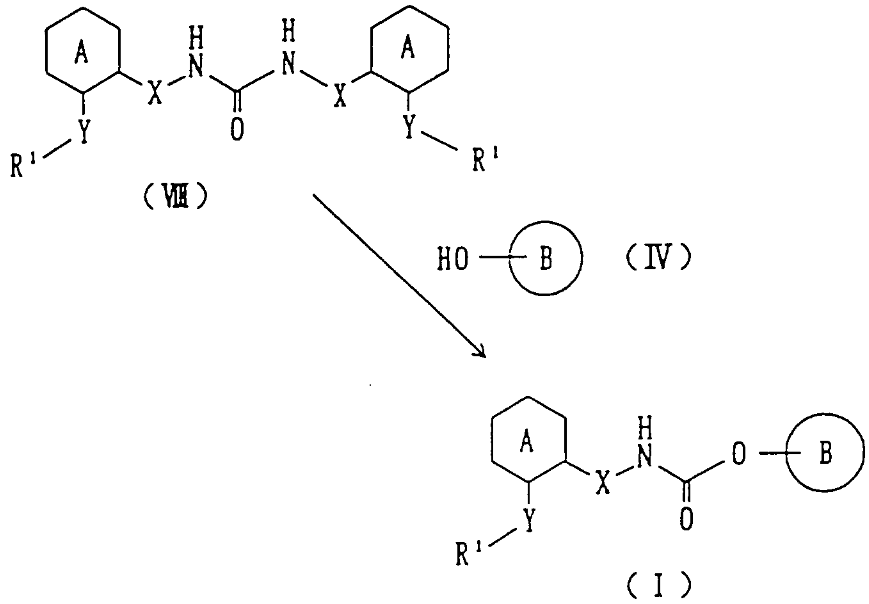
(VIII)
(IV)
(I)

(In the above reaction formula, A ring, B ring, $R^1$, X and Y are as defined in the foregoing.)

The compound (I) of the present invention can be obtained by allowing a compound represented by the general formula (VIII) to react with an alcohol compound represented by the general formula (IV).

This reaction is carried out by stirring the compound (VIII) and the compound (IV) in an amount corresponding to the reaction in the aforementioned inert solvent with heating.

In order to accelerate the reaction, it is desirable to add a base (e.g., sodium, sodium hydride or the like).

Fourth Preparation Method

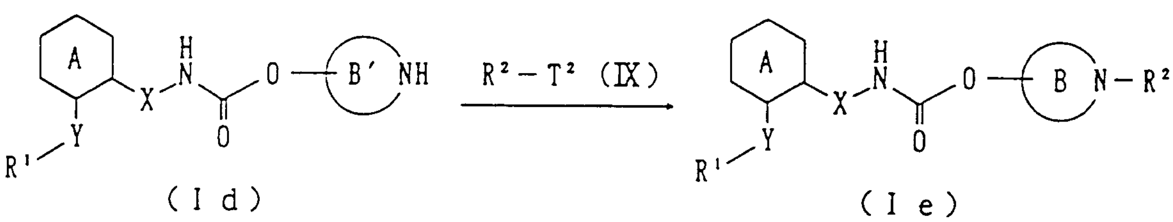

(Id)
(Ie)

(In the above reaction formula, A ring, $R^1$, $R^2$, X and Y are as defined in the foregoing, B' ring is a ring in which Z in the B ring is NH, and $T^2$ is the aforementioned leaving group or a formyl group.)

The fourth preparation method is an N-alkylation reaction to prepare a compound which has a lower alkyl group, a lower alkenyl group, a lower alkynyl group or an aralkyl group as the substituent $R^2$ of its B ring (this reaction is called

N-alkylation reaction hereinafter).

The compound (Ie) of the present invention can be obtained by allowing a compound represented by the general formula (Id) to react with a compound represented by the general formula (IX).

This reaction can be effected in accordance with the usual N-alkylation reaction.

(1) A case in which the compound (IX) is an alkyl halide or an alkyl sulfonate: This reaction is carried out by stirring the compound (Id) and the compound (IX) in an amount corresponding to the reaction in the aforementioned inert solvent at ice-cooled temperature to heating temperature. In order to accelerate the reaction, it is desirable to add a base (e.g., an inorganic base such as potassium carbonate, sodium carbonate or the like or an organic base such as triethylamine or the like).

(2) A case in which the compound (IX) is an aldehyde: This reaction is a dehydration condensation reaction in which the compound (Id) in an amount corresponding to the reaction is allowed to react with an aldehyde, $R^2$-CHO (IX), and a reducing agent. By this alkylation, not the compound N-$R^2$ shown in the aforementioned reaction formula but a compound N-$CH_2$-$R^2$ is formed.

As the reducing agent, sodium borohydride, sodium cyanoborohydride, sodium triacetoxy borohydride or the like is used. This reaction is carried out by stirring the reaction materials in an alcohol or the aforementioned inert solvent at ice-cooled temperature to heating temperature (under reflux). Alternatively, it may be effected by carrying out catalytic hydrogenation under normal or high pressure in the presence of a catalyst such as palladium-carbon, platinum oxide or the like.

Fifth Preparation Method

A member of the compound of the present invention in which $R^2$ of the B ring is a hydrogen atom is prepared from another member of the compound of the present invention in which $R^2$ is an aralkyl group which may have a substituent or a lower alkyl group substituted with a heterocyclic group which has 1 or 2 hetero atoms, which may have a substituent and which may be fused. In one preparation method, the compound of the present invention whose $R^2$ is the aforementioned substituent and a chloroformic acid ester (e.g., 1-chloroethyl chloroformate) in an amount corresponding to the reaction are stirred in an inert solvent at room temperature to heating temperature and then subjected to generally used solvolysis. In another preparation method, the compound of the present invention in which the aforementioned $R^2$ is a benzyl group which may have a substituent is subjected to usual hydrogenation reaction in the presence of a catalyst (such as palladium-carbon, platinum oxide, palladium hydroxide or the like).

Sixth Preparation Method.

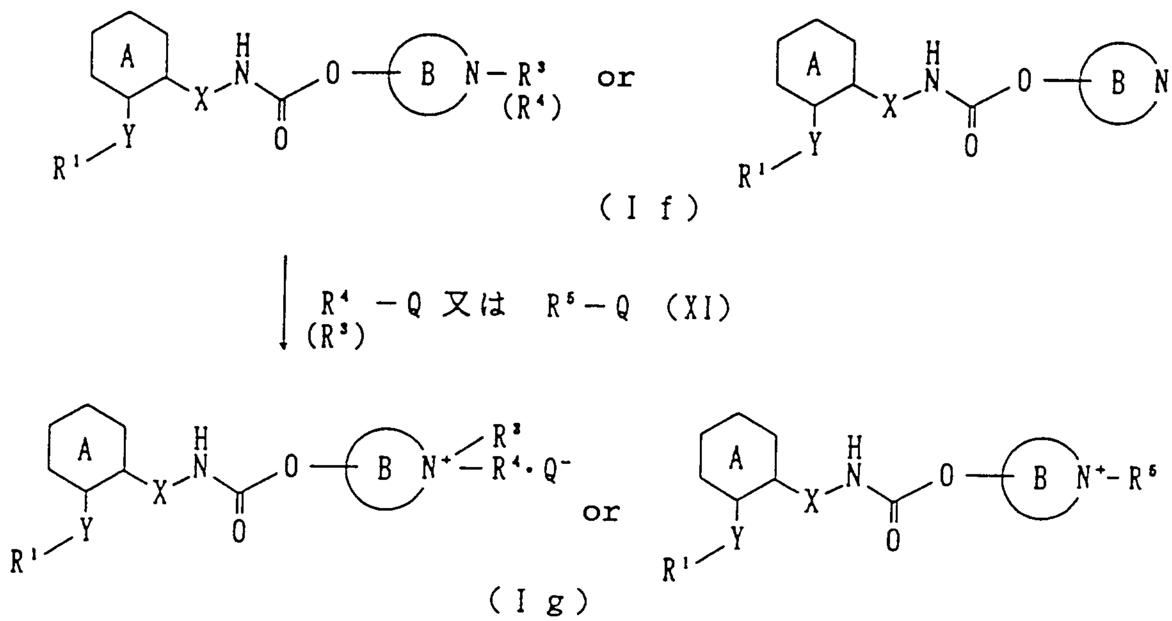


or

(I f)

or

(I g)

(In the above reaction formula, A ring, B ring, $R^1$, $R^3$, $R^4$, $R^5$, X, Y and Q are as defined in the foregoing.)

In this preparation method, a quaternary ammonium compound (Ig), namely a compound in which the B ring of the compound of the present invention has a group of the formula

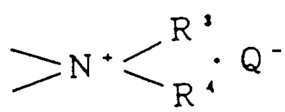

or the formula $\geqslant N^+-R^5 \cdot Q^-$ as Z or Z', is prepared by the N-alkylation reaction of a compound (If) in which the amine of the B ring of the present invention is a secondary or tertiary amine. In this connection, the compound (If) is a compound represented by the general formula ($Ia^1$), ($Ib^1$) or ($Ic^1$) (provided that q = 0), and the compound (Ig) is a compound represented by the general formula ($Ia^2$), ($Ib^2$) or ($Ic^2$). When the compound (If) is a secondary amine, the quaternary ammonium compound (Ig) is obtained by allowing 1 mol of the compound (If) to react with at least 2 mols of an alkylation agent (XI).

This reaction is carried out by stirring the compound (If) and an alkylation agent (XI) in an amount corresponding to the reaction in an inert solvent such as dimethylformamide, chloroform, benzene, 2-butanone, acetone, tetrahydrofuran or the like, at ice-cooled temperature to room temperature or with heating as occasion demands.

Examples of the alkylation agent include a lower alkyl halide, a lower alkyl-trifluoromethanesulfonate, a lower alkyl-p-toluenesulfonate, a lower alkyl-methanesulfonate and the like. Preferred of these is a lower alkyl halide.

Seventh Preparation Method

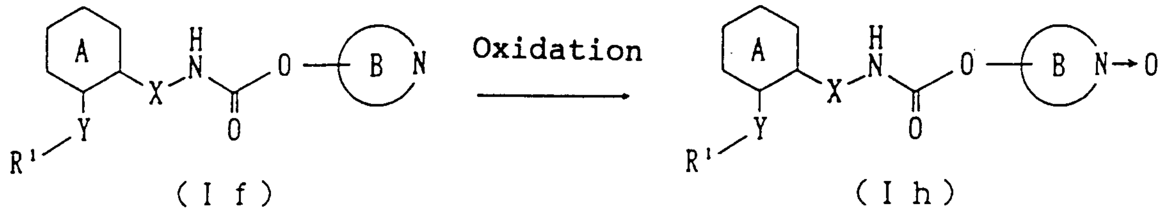


(I f) (I h)

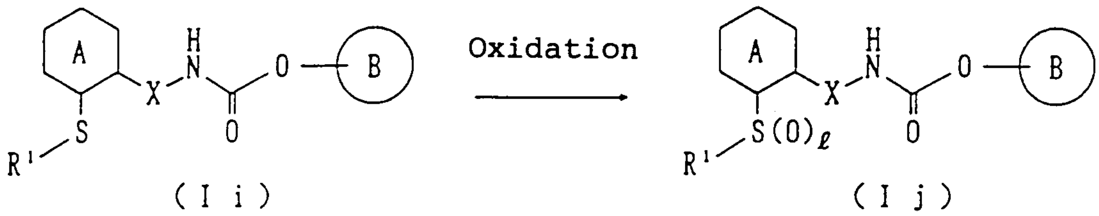


(I i) (I j)

(In the above reaction formula, A ring, B ring, ℓ, $R^1$, X and Y are as defined in the foregoing.)

In this preparation method, an N-oxide compound (Ih) or a sulfoxide or sulfone compound (Ij) is obtained by oxidizing a compound in which the amine of the B ring of the compound of the present invention is a tertiary amine (If) or a sulfide compound (Ii).

This reaction is carried out by stirring the compound (If) or (Ii) and its corresponding or excess amount of an oxidizing agent in an inert solvent such as chloroform, dichloromethane or the like, an alcohol such as methanol, ethanol or the like, water or a mixed solvent thereof, at ice-cooled temperature to room temperature or at heating temperature as occasion demands.

Examples of the oxidizing agent include organic peracids such as m-chloroperbenzoic acid and the like, sodium periodate, hydrogen peroxide and the like.

Eighth Preparation Method

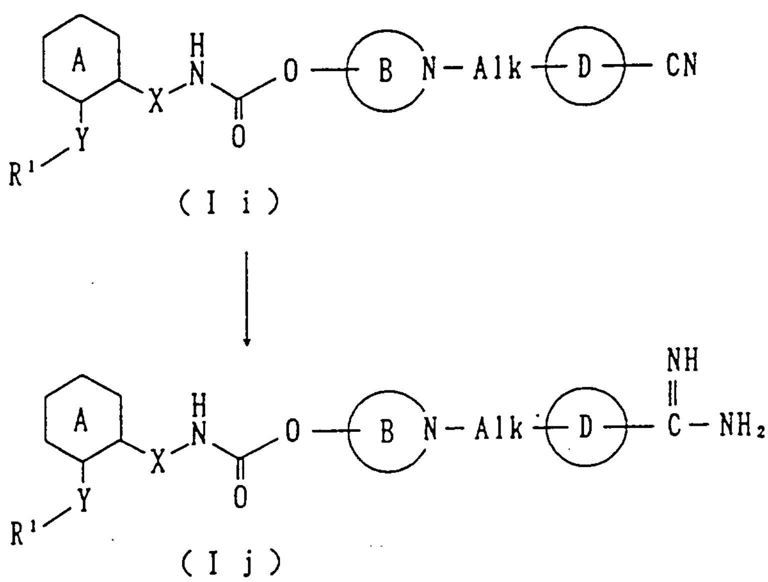

(I i)

(I j)

(In the above reaction formula, A ring, B ring, $R^1$, X and Y are as defined in the foregoing, Alk means the lower alkyl moiety of the aralkyl group of $R^2$ or $R^3$ and D ring means the aryl moiety.)

This preparation method is for use in the preparation of a compound which has amidino group as the substituent of the aryl moiety of aralkyl group which may have a substituent. That is, a compound (Ij) having amidino group can be synthesized by the following method (i), (ii) or (iii).

(i) A method in which nitrile is converted into imidate and then condensed with amine

A nitrile compound (Ii) is allowed to react with an alcohol such as methanol, ethanol or the like at -40°C to 0°C in the presence of hydrogen chloride gas to convert it into imidate which is subsequently allowed to react with ammonia, or an amine or an ammonium salt such as ammonium carbonate, ammonium chloride, ammonium acetate or the like. As the solvent, methanol, ethanol, acetone, tetrahydrofuran or the like may be used.

(ii) A method in which nitrile is converted into thioamide and then into thioimidate which is then condensed with amine

The nitrile compound (Ii) is allowed to react with hydrogen sulfide in the presence of an organic base such as methylamine, triethylamine, pyridine, picoline or the like, thereby obtaining a thioamide compound. The thioamide compound can also be obtained by allowing O,O-diethyl dithiophosphate to react with the nitrile compound (Ii) in the presence of hydrogen chloride.

The thioamide compound is allowed to react with a lower alkyl halide such as methyl iodide, ethyl iodide or the like to convert it into thioimidate which is subsequently allowed to react with ammonia, or an amine or an ammonium salt such as ammonium carbonate, ammonium chloride, ammonium acetate or the like. As the solvent, methanol, ethanol, acetone, tetrahydrofuran, ethyl acetate or the like may be used.

(iii) A method in which amine, ammonium salt, metal amide and Grignard's reagent are directly added to nitrile

In an appropriate solvent or with no solvent, to the nitrile compound (Ii) are added various reagents such as ammonia, ammonium chloride and ammonia, ammonium thiocyanate, alkyl ammonium thiocyanate, $MeAl(Cl)NH_2$, $NaNH_2$, $(CH_3)_2NMgBr$ and the like, thereby effecting synthesis of the compound of interest. As the solvent, chloroform, methanol, ethanol, acetone, tetrahydrofuran, toluene, dimethylformamide or the like may be used. In some cases, the reaction may be markedly accelerated when a base such as sodium hydride or the like or an acid such as aluminum chloride, p-toluenesulfonic acid or the like is used as a catalyst. The reaction can be carried out at cooling temperature to room temperature or at heating temperature.

(Other Preparation Methods)

A member of the compound of the present invention in which the aryl moiety of the $R^2$ aralkyl group which may have a substituent is an aminophenyl group is prepared from another member of the compound of the present invention in which said aryl moiety is nitrophenyl group. In one preparation method, the compound of interest is obtained by subjecting the compound of the present invention in which said aryl moiety is nitrophenyl group to hydrogenation reaction at room temperature or a heating temperature in the presence of a catalyst (for example, Raney nickel, palladium-carbon, palladium, platinum oxide, palladium hydroxide or the like). In another preparation method, the compound of interest is obtained by subjecting the compound of the present invention in which said aryl moiety is a nitrophenyl group to reduction which is carried out in an active solvent in the presence of a reaction corresponding amount of a metal such as iron powder, tin, zinc or the like at ice-cooled temperature to room temperature or at a heating temperature as occasion demands.

A member of the compound of the present invention in which Y is methylene group substituted with hydroxyl group is produced from another compound of the present invention in which Y is carbonyl group. That is, it can be obtained by subjecting the carbonyl group-containing compound of the present invention to a reduction reaction which is carried out in an active solvent such as an alcohol or the like in the presence of a reducing agent such as sodium borohydride or the like at a cooling temperature to room temperature.

When the compound of the present invention is produced, protection of functional groups will be required in certain cases. In that case, it can be prepared in the usual way by adding appropriate deprotection procedures.

The resulting compound of the present invention is isolated and purified as its free form or a salt thereof by carrying out generally used salt forming treatment. Isolation and purification are carried out by employing usual chemical procedures such as extraction, concentration, evaporation, crystallization, filtration, recrystallization, various types of chromatography and the like.

INDUSTRIAL APPLICABILITY

The compounds of the present invention have affinity and selectivity for muscarinic $M_3$ receptors and are useful as $M_3$ receptor antagonists for use in the prevention or treatment of various $M_3$ receptor-related diseases, particularly respiratory diseases such as chronic obstructive pulmonary disease, chronic bronchitis, asthma, rhinitis and the like, urinary diseases such as urinary incontinence, pollakiuria and the like in neurogenic pollakiuria, neurogenic bladder, nocturnal enuresis, unstable bladder, cystospasm, chronic cystitis and the like and gastrointestinal diseases such as

irritable bowel syndrome, spastic colitis, diverticulitis and the like.

Particularly, the compounds of the present invention have higher selectivity for the $M_3$ receptors which exist in smooth muscles, gland tissues and the like, over the $M_2$ receptors which exist in the heart and the like, so that they are highly useful as an $M_3$ receptor antagonists which hardly cause side effects in the heart and the like, especially as preventive or therapeutic drugs of chronic bronchitis, asthma, rhinitis, the aforementioned urinary incontinence and pollakiuria, irritable bowel syndrome, chronic obstructive pulmonary disease and the like.

Affinity of the compound of the present invention for muscarinic receptors and its antagonism were confirmed by the following tests.

Muscarinic receptor binding test

a. Preparation of membranes

Heart and submandibular gland were separately excised from each of Wistar male rats of 200 to 350 g in weight (purchased from Nippon SLC), mixed with 5 volumes of 20 mM HEPES buffer containing 100 mM sodium chloride and 10 mM magnesium chloride (pH 7.5, to be referred to as HEPES buffer hereinafter) and homogenized under ice-cooling. This was filtered through a gauze, subjected to 10 minutes of ultracentrifugation at 50,000 × g and at 4°C, and the resulting pellets was suspended in HEPES buffer and again subjected to 10 minutes of ultracentrifugation at 50,000 × g and at 4°C. The resulting pellets were suspended in HEPES buffer and preserved at -80°C. The following tests were carried by thawing out the resulting samples when used.

b. Muscarinic $M_2$ receptor binding test

This test was carried out by modifying the method of Doods *et al*. (*J. Pharmacol. Exp. Ther.*, 242, 257 - 262, 1987). Heart membranes, $[^3H]$-quinuclidinyl benzilate and each compound to be tested were incubated in 0.5 ml of HEPES buffer at 25°C for 45 minutes and then mixed with 5 ml of HEPES buffer and filtered using suction through a glass filter (Whatman GF/B), and the filter was washed three times with 5 ml of HEPES buffer. Radioactivity of $[^3H]$-quinuclidinyl benzilate absorbed in the filter was measured with a liquid scintillation counter. In this case, non-specific binding was determined by adding 1 μM of atropine. Affinity of the compounds of the present invention for muscarinic $M_2$ receptors was obtained as a dissociation constant (Ki) calculated from the concentration of each test compound which inhibits 50% of binding of the labeled ligand $[^3H]$-quinuclidinyl benzilate ($IC_{50}$) in accordance with the method of Cheng and Prusoff (*Biochem. Pharmacol.*, 22, 3099, 1073).

c. Muscarinic $M_3$ receptor binding test

This test was carried out by the same method of the just described in the muscarinic $M_2$ receptor binding test, except that submandibular gland membranes were used and $[^3H]$-N-methylscopolamine was used as the labeled ligand.

Results: Table 1 shows results of the above tests carried out using typical examples of the compound of the present invention. As is evident from Table 1, the compounds (I) of the present invention have Ki values of $10^{-8}$ to $10^{-10}$ M for $M_3$ receptors, thus showing higher binding ability than the case of $M_2$ more than 10-fold.

Table 1

| Example No. | Ki (nM) | | $M_2/M_3$ |
|---|---|---|---|
| | $M_2$ binding Test | $M_3$ binding Test | |
| 1 | 25.9 | 0.94 | 27.6 |
| 2 | 100 | 4.89 | 20.4 |
| 3 | 14.4 | 0.56 | 25.7 |
| 4 | 5.89 | 0.39 | 15.2 |
| 7 | 14.7 | 0.57 | 25.8 |
| Comparative Example | | | |
| Compound A | 400 | 95 | 4.2 |
| Atropine | 0.98 | 0.39 | 2.5 |
| Compound A: compound (I) disclosed in GB 2249093<br>Atropine: Merck Index (11th ed., p.138) | | | |

Muscarinic receptor antagonism test (*in vivo*)

a. Test on bronchospasm in guinea pig

This test was carried out in accordance with the method of *Konzett et al.* (*Arch. Exp. Path. Pharmak.*, 195, 71 - 74, 1940). Under urethane anesthesia (1.5 g/kg, i.p.), a cannula was inserted into the trachea of a Hartley male guinea pig (400 - 700 g) and connected to a ventilator. In order to stop spontaneous respiration, gallamine was administered (2 mg/kg, i.v.). Bronchospasm was measured using Bronchospasm Transducer manufactured by Ugo Basile. After twice inducing reproducible bronchospasm by administration of methacholine (5 µg/kg, i.v.), each compound to be tested was administered through a catheter indwelt in the external jugular vein and, 3 minutes thereafter, bronchospasm was again induced with methacholine. By calculating inhibition ratio of bronchospasm by administration of each compound to be tested, a dose of the compound which inhibited 50% of bronchospasm before its administration was calculated as an $ID_{50}$ value.

Results: The values of typical compounds are shown in Table 2. The compound (I) of the present invention showed excellent $ID_{50}$ values.

Table 2

| Example No. | Bronchospasm Test $ID_{50}$ (mg/kg i.v.) |
|---|---|
| 1 | 0.0045 |
| 3 | 0.0038 |
| 4 | 0.0082 |
| 7 | 0.0054 |
| Atropine | 0.00080 |

b. Test on rhythmic bladder contraction in rat

Under urethane anesthesia (1.0 g/kg, s.c.), bilateral ureters of each of Wistar female rats (140 - 200 g) were ligated just distal to the kidneys. Rhythmic bladder contractions were induced by injecting 0.5 to 1 ml of physiological saline into the bladder through a catheter transurethrally indwelt in the bladder, and intravesical pressure was measured using a pressure transducer. After observing stable rhythmic contractions for 5 minutes or more, each compound to be tested was cumulatively administered into the external jugular vein, and intravesical pressure was measured from 5 to 10 minutes after administration. By calculating inhibition ratio of peak bladder contractions by administration of the compound to be tested, a dose of the compound which inhibited 30% of the peak bladder contractions before its administration was calculated as an $ED_{30}$ value.

Results of typical compounds are shown in Table 3. The compound of the present invention showed excellent $ED_{30}$ values.

c. Test on salivary secretion in rat

Male Wistar rats (6 weeks of age) were anesthetized with urethane (0.8 g/kg, i.p.). Fifteen minutes after the administration of each compound to be tested (a solvent for control group), oxotremorine was administered at a dose of 0.8 µmol/kg. Each drug was administered through the femural vein. Saliva secreted during 5 minutes after the administration of oxotremorine was recovered and weighed. By calculating inhibition ratio of the amount of secreted saliva in the control group, a dose of the compound which inhibited 50% of the amount of secreted saliva in the control group was calculated as an $ID_{50}$ value.

Results: The value of typical compounds are shown in Table 3. The $ID_{50}$ values of atropine and oxybutynin were similar to the $ED_{30}$ values of the aforementioned rat rhythmic bladder contraction test, while $ID_{50}$ values of the compounds of the present invention were 5 to 10 times lower than the aforementioned $ED_{30}$ values, thus showing their relatively weak action on salivation.

Table 3

| Example No. | Rhythmic Bladder Contraction Test $ED_{30}$ (mg/kg i.v.) | Salivary Secretion Test $ED_{50}$ (mg/kg i.v.) |
|---|---|---|
| 2 | 0.018 | 0.11 |
| 4 | 0.014 | 0.10 |
| 15 | 0.058 | 0.36 |
| 61 | 0.029 | 0.14 |
| Atropine | 0.009 | 0.0084 |
| Oxybutynin | 0.24 | 0.18 |

As the results of the above muscarinic $M_3$ receptor affinity test and muscarinic $M_3$ receptor antagonism test (*in vivo*), the compounds (I) of the present invention showed selective and high affinity for $M_3$ receptors and excellent muscarinic $M_3$ receptor antagonism against *in vivo* rhythmic bladder contraction and bronchospasm. In consequence, it was confirmed that the compounds (I) of the present invention antagonize the muscarinic $M_3$ receptor selectively. In addition, it showed less side effects such as dry mouth and the like which are common with conventional anticholinergic drugs.

A pharmaceutical composition which contains at least one member of the compound of the present invention or salts thereof as the active ingredient is prepared using carriers, excipients and other additives commonly used for the preparation of medicines.

As the carriers and excipients for pharmaceutical preparation use, solid or liquid nontoxic pharmaceutical materials may be used. Their illustrative examples include lactose, magnesium stearate, starch, talc, gelatin, agar, pectin, acacia, olive oil, sesame oil, cacao butter, ethylene glycol and other generally used materials.

The preparation of the present invention may be administered either by oral administration in the dosage form of tablets, pills, capsules, granules, powders, solutions and the like or by parenteral administration in the dosage form of injections such as intravenous injection, intramuscular injection, or the like, suppositories, transdermal absorption preparations, inhalations, intravesical injections and the like. Though the dose is optionally decided in individual cases, taking symptoms of the disease and age, sex, and the like of each patient into consideration, it may be within the range of generally from 0.05 to 100 mg per day per adult in the case of oral administration, and the daily dose may be used once a day or by dividing it into 2 to 4 doses a day. When the preparation is intravenously administered depending on the symptoms, it may be administered generally within the range of from 0.001 mg to 10 mg per adult per day, once a day or by dividing the daily dose into several doses a day.

The solid composition of the present invention for oral administration use may be used in the dosage form of tablets, powders, granules and the like. In such a solid composition, at least one active ingredient is mixed with at least one inert diluent such as lactose, mannitol, glucose, hydroxypropylcellulose, microcrystalline cellulose, starch, polyvinylpyrrolidone, magnesium metasilicate aluminate or the like. In the usual way, the composition may contain additives other than the inert diluent, for example, lubricants such as magnesium stearate and the like, disintegrators such as calcium carboxymethylcellulose and the like, stabilizers such as lactose and the like, and solubilizers such as glutamic acid, aspartic acid and the like. As occasion demands, tablets or pills may be coated with a gastric or enteric film such as of sucrose, gelatin, hydroxypropylcellulose, hydroxypropylmethylcellulose phthalate or the like.

The liquid composition used for oral administration includes pharmaceutically acceptable emulsions, solutions, suspensions, syrups, elixirs and the like and contains generally used inert diluents such as purified water, ethanol and the like. In addition to the inert diluent, this composition may also contain auxiliary agents such as a moistening agent, a suspending agent and the like, sweeteners, flavors, aromatic agents, antiseptics and the like.

Injections used for parenteral administration include aseptic aqueous or non-aqueous solutions, suspensions and emulsions. Examples of the aqueous solutions and suspensions include distilled water for injection use and physiological saline. Examples of the non-aqueous solutions and suspensions include propylene glycol, polyethylene glycol, plant oils such as olive oil and the like, alcohols such as ethanol and the like and Polysorbate 80. Such compositions may further contain auxiliary agents such as antiseptics, moistening agents, emulsifying agents, dispersing agents, stabilizing agents (e.g., lactose), solution aids (e.g., glutamic acid and aspartic acid) and the like. These compositions are sterilized for example by bacterial filtration, blending of bactericides or irradiation. Alternatively, a sterile solid composition prepared in advance may be used by dissolving it in sterile water or a sterile solvent for injection use prior to its use.

BEST MODE FOR CARRYING OUT THE INVENTION

Examples of the present invention are given below by way of illustration. The compounds of the present invention are not limited to the compounds described in the following examples, and not only the compounds represented by the aforementioned general formula (I) but also salts thereof, hydrates thereof, solvates thereof, geometrical and optical isomers thereof and polymorphic forms thereof are all included in the present invention.

Reference Example 1

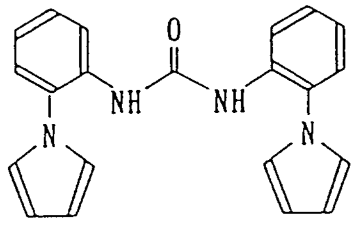

Under ice-cooling, 3.28 g of methyl chloroformate was added dropwise to 50 ml of a dichloromethane solution containing 5.02 g of 1-(2-aminophenyl)pyrrole and 3.83 g of triethylamine for 5 minutes, and the resulting mixture was stirred overnight at room temperature. Under ice-cooling, 3.83 g of triethylamine was added, and 3.28 g of methyl chloroformate was added dropwise thereto for 5 minutes and then the resulting mixture was stirred at room temperature for 6 hours. Again, under ice-cooling, 3.83 g of triethylamine was added, and 3.28 g of methyl chloroformate was added dropwise thereto for 5 minutes. Then, the resulting mixture was stirred overnight at room temperature. The reaction mixture was mixed with 100 ml of water, the resulting organic layer was separated and dried over anhydrous magnesium sulfate and then the solvent was removed under reduced pressure. The resulting residue was purified by silica gel column chromatography (hexane/ethyl acetate = 4/1) to give 1.34 g of 1,3-bis[2-(1H-pyrrol-1-yl)phenyl]urea as colorless solid.

Infrared absorption spectrum $\nu_{max}$ (KBr) $cm^{-1}$: 3320, 1662, 1550
Nuclear magnetic resonance spectrum ($CDCl_3$, TMS internal standard)
δ: 6.30 (4 H, dd, J = 2.5 Hz, 1.8 Hz), 6.46 (2 H, s), 6.71 (4 H, dd, J = 2.4 Hz, 1.9 Hz), 7.10 - 7.16 (2 H, m), 7.20 - 7.26 (2 H, m), 7.32 - 7.38 (2 H, m), 8.04 (2 H, d, J = 7.3 Hz)

The following compound was obtained in the same manner as described in Reference Example 1.

Reference Example 2

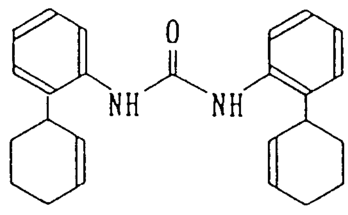

1,3-Bis[2-(2-cyclohexen-1-yl)phenyl]urea Starting compounds: 2-(2-cyclohexen-1-yl)aniline, ethyl chloroformate

Mass spectrum data (m/z, FAB): 373 ($M^+$ + 1)
Nuclear magnetic resonance spectrum (DMSO-$d_6$, TMS internal standard)
δ: 1.35 - 1.50 (2 H, m), 1.50 - 1.75 (4 H, m), 1.90 - 2.20 (6 H, m), 3.65 - 3.75 (2 H, m), 5.63 (2 H, dm, J = 10.3 Hz), 5.94 (2 H, dm, J = 10.3 Hz), 7.00 - 7.20 (6 H, m), 7.55 - 7.65 (2 H, m), 8.18 (1 H, s)

Example 1

A 2.89 g portion of diphenylphosphoryl azide was added dropwise at room temperature to 50 ml of a toluene solution containing 1.98 g of 2-biphenylcarboxylic acid and 1.11 g of triethylamine, followed by stirring at 60°C for 1.5 hours. Next, 1.27 g of 3-quinuclidinol was added, followed by heating under reflux for 6 hours. The reaction mixture was cooled to room temperature and washed with water, saturated sodium bicarbonate aqueous solution, water and brine, and the resulting organic layer was dried over anhydrous sodium sulfate. The solvent was removed under reduced pressure. The resulting residue was purified by silica gel column chromatography (chloroform/methanol = 9/1) to give 2.47 g of 3-quinuclidinyl N-(2-biphenylyl)carbamate as colorless solid. This was dissolved in 30 ml of ethanol and mixed with 4 ml of 4 N hydrogen chloride-dioxane solution, the solvent was removed under reduced pressure and then the residual solid was recrystallized from isopropanol-diethyl ether to give 2.23 g of 3-quinuclidinyl N-(2-biphenylyl)carbamate monohydrochloride as colorless crystals.

Melting point: 137 - 138°C (i-PrOH-$Et_2O$)

| Elemental analysis (for $C_{20}H_{23}N_2O_2Cl \cdot 0.8H_2O$): | | | | |
|---|---|---|---|---|
| | C (%) | H (%) | N (%) | Cl (%) |
| Calcd. | 64.35 | 6.64 | 7.50 | 9.50 |
| Found | 64.38 | 6.62 | 7.36 | 9.50 |

Example 2

A 30 mg portion of sodium hydride (60% in mineral oil) was added to a solution of 30 ml toluene and 1 ml dimethylformamide containing 1.00 g of 1,3-bis[2-(1H-pyrrol-1-yl)phenyl]urea and 1.05 g of 3-quinuclidinol, and the resulting mixture was heated under reflux for 6 hours. After cooling, this was diluted with 30 ml of ethyl acetate, then washed with water and the organic layer was dried over anhydrous magnesium sulfate. After removal of the solvent under reduced pressure, the resulting residue was purified by silica gel column chromatography (chloroform/methanol = 9/1) to give 0.58 g of 3-quinuclidinyl N-[2-(1H-pyrrol-1-yl)phenyl]carbamate as pale yellow oil. This was dissolved in 5 ml of ethanol and mixed with 1 ml of 4 N hydrogen chloride-ethyl acetate solution and then the solvent was removed under reduced pressure. The resulting residue was recrystallized from ethanol-diethyl ether to give 0.35 g of 3-quinuclidinyl N-[2-(1H-pyrrol-1-yl)phenyl]carbamate monohydrochloride as colorless crystals.

Melting point: 183 - 185°C (EtOH-$Et_2O$)

| Elemental analysis (for $C_{18}H_{22}N_3O_2Cl \cdot 0.2H_2O$): | | | | |
|---|---|---|---|---|
| | C (%) | H (%) | N (%) | Cl (%) |
| Calcd. | 61.52 | 6.42 | 11.96 | 10.09 |
| Found | 61.59 | 6.37 | 11.95 | 10.36 |

Example 3

A 0.16 ml portion of methyl iodide was added to 5 ml of a 2-butanone solution containing 0.46 g of 3-quinuclidinyl N-(2-biphenylyl)carbamate, and the mixture was stirred for 5.5 hours at room temperature and then overnight below 5°C. The resulting precipitated crystals were collected by filtration and washed with diethyl ether to give 0.58 g of 3-[[N-(2-biphenylyl)carbamoyl]oxy]-1-methylquinuclidinium iodide as pale yellow crystals.

Melting point: 220 - 221°C (2-butanone)

| Elemental analysis (for $C_{21}H_{25}N_2O_2I$): | | | | |
|---|---|---|---|---|
| | C (%) | H (%) | N (%) | I (%) |
| Calcd. | 54.32 | 5.43 | 6.03 | 27.33 |
| Found | 54.16 | 5.43 | 5.89 | 27.36 |

Example 4

A 3.10 g portion of diphenylphosphoryl azide was added dropwise at room temperature to 30 ml of a toluene solution containing 2.00 g of 2-biphenylcarboxylic acid and 1.22 g of triethylamine, and the reaction mixture was stirred at 90°C for 20 minutes. And then, this was mixed with 10 ml of a dimethylformamide solution containing 4.00 g of 4-quinuclidinol • p-toluenesulfonate and 1.50 g of triethylamine, followed by heating under reflux for 3 hours. After air-cooling, 30 ml of ethyl acetate was added, followed by washing with water twice. After additional washing with a saturated sodium bicarbonate aqueous solution, the resulting organic layer was dried over anhydrous magnesium sulfate, and the solvent was removed under reduced pressure. The resulting residue was purified by silica gel column chromatography (chloroform/methanol = 29/1 → 9/1) to give 1.82 g of 4-quinuclidinyl N-(2-biphenylyl)carbamate as light brown solid. This was dissolved in 30 ml of ethanol and mixed with 3 ml of a 4 N hydrogen chloride-ethyl acetate solution and then the solvent was removed under reduced pressure. The resulting solid was recrystallized from ethanol-diethyl ether to give 0.91 g of 4-quinuclidinyl N-(2-biphenylyl)carbamate monohydrochloride as colorless crystals.

Melting point: 225 - 226°C (EtOH-$Et_2O$)

| Elemental analysis (for $C_{20}H_{23}N_2O_2Cl \cdot 0.2H_2O$): | | | | |
|---|---|---|---|---|
| | C (%) | H (%) | N (%) | Cl (%) |
| Calcd. | 66.27 | 6.51 | 7.73 | 9.78 |
| Found | 66.25 | 6.53 | 7.73 | 9.76 |

Example 5

A 0.11 ml portion of methyl iodide was added to 5 ml of a 2-butanone solution containing 0.57 g of 4-quinuclidinyl N-(2-biphenylyl)carbamate, and the reaction mixture was stirred at room temperature overnight. After removal of the solvent under reduced pressure, the resulting residue was solidified by adding acetone and then recrystallized from acetonitrile to give 0.49 g of 4-[[N-(2-biphenylyl)carbamoyl]oxy]-1-methylquinuclidinium iodide as colorless crystals.

Melting point: 123 - 125°C ($CH_3CN$)

| Elemental analysis (for $C_{21}H_{25}N_2O_2I \cdot 1.8H_2O$): | | | | |
|---|---|---|---|---|
| | C (%) | H (%) | N (%) | I (%) |
| Calcd. | 50.77 | 5.80 | 5.64 | 25.55 |
| Found | 50.39 | 5.48 | 5.64 | 25.76 |

Example 6

With stirring, methyl bromide was bubbled into 10 ml of a 2-butanone solution containing 0.97 g of 4-quinuclidinyl

N-(2-biphenylyl)carbamate for 20 minutes. After additional 1 hour of stirring at room temperature, the precipitated crystals were collected by filtration and washed with 2-butanone to give 1.06 g of 4-[[N-(2-biphenylyl)carbamoyl]oxy]-1-methylquinuclidinium bromide as colorless crystals.

Melting point: 243 - 244°C (2-butanone)

| Elemental analysis (for $C_{21}H_{25}N_2O_2Br$): | | | | |
|---|---|---|---|---|
| | C (%) | H (%) | N (%) | Br (%) |
| Calcd. | 60.44 | 6.04 | 6.71 | 19.15 |
| Found | 60.34 | 6.14 | 6.61 | 19.01 |

Example 7

A 0.11 ml portion of methyl iodide was added dropwise at room temperature to a suspension of 0.50 g of 4-piperidyl N-(2-biphenylyl)carbamate and 0.23 g of potassium carbonate in acetonitrile, and the reaction mixture was stirred at room temperature for 23 hours. Insoluble matter was removed by filtration and the solvent was removed under reduced pressure. The resulting residue was purified by silica gel column chromatography (chloroform/methanol = 20/1 → 5/1) to give 0.36 g of 4-[[N-(2-biphenylyl)carbamoyl]oxy]-1,1-dimethylpiperidinium iodide as colorless solid. This was recrystallized from methanol-diethyl ether to give 0.13 g of 4-[[N-(2-biphenylyl)carbamoyl]oxy]-1,1-dimethylpiperidinium iodide as colorless crystals.

Melting point: 223 - 224°C (MeOH-$Et_2O$)

| Elemental analysis (for $C_{20}H_{25}N_2O_2I \cdot 0.3H_2O$): | | | | |
|---|---|---|---|---|
| | C (%) | H (%) | N (%) | Cl (%) |
| Calcd. | 52.48 | 5.64 | 6.12 | 27.72 |
| Found | 52.12 | 5.46 | 6.02 | 28.05 |

The following compounds of Examples 8 to 10 were prepared in the same manner as the procedure of Example 1.

Example 8

3-Quinuclidinyl N-(2-benzylphenyl)carbamate monohydrobromide

Starting compound: 2-benzylbenzoic acid

Melting point: 170 - 172°C ($CH_3CN$-i-PrOH)

| Elemental analysis (for $C_{21}H_{25}N_2O_2Br$): | | | | |
|---|---|---|---|---|
| | C (%) | H (%) | N (%) | Br (%) |
| Calcd. | 60.44 | 6.04 | 6.71 | 19.15 |
| Found | 60.33 | 6.02 | 6.71 | 19.06 |

Example 9

3-Quinuclidinyl N-(2-phenyl-3-pyridyl)carbamate monooxalate

Starting compound: 2-phenylnicotinic acid

Form: colorless amorphous

| Elemental analysis (for $C_{21}H_{23}N_3O_6 \cdot 1.75H_2O$): | | | |
|---|---|---|---|
| | C (%) | H (%) | N (%) |
| Calcd. | 56.69 | 6.00 | 9.44 |
| Found | 56.88 | 5.74 | 9.12 |

Mass spectrum data (m/z, FAB): 324 ($M^+$ + 1)
Infrared absorption spectrum $\nu_{max}$ (KBr) $cm^{-1}$: 1724, 1636, 1442, 1246

Example 10

3-Quinuclidinyl N-(2-biphenylylmethyl)carbamate monooxalate

Starting compound: 2-biphenylacetic acid

Melting point: 73 - 76°C (EtOH-i-$Pr_2O$)

| Elemental analysis (for $C_{23}H_{26}N_2O_6 \cdot 0.75H_2O$): | | | |
|---|---|---|---|
| | C (%) | H (%) | N (%) |
| Calcd. | 62.79 | 6.30 | 6.37 |
| Found | 62.67 | 6.43 | 6.06 |

Example 11

A 8.67 g portion of diphenylphosphoryl azide was added to 150 ml of a toluene solution containing 5.95 g of 2-phenylbenzoic acid and 3.34 g of triethylamine, and the reaction mixture was stirred at 60°C for 1.5 hours. Then, 6.51 g of 1-benzyl-4-piperidinol was added, and the mixture was stirred under reflux for 6 hours. The reaction solution was cooled to room temperature, successively washed with water, a saturated sodium bicarbonate aqueous solution, water and brine and then dried over anhydrous sodium sulfate. The solvent was removed under reduced pressure. The resulting residue was purified by silica gel column chromatography (chloroform/methanol = 100/1 → 50/1) to give 11.27 g of 1-benzyl-4-piperidyl N-(2-biphenylyl)carbamate. A 0.54 g portion thereof was dissolved in 20 ml of ethanol, 0.5 ml of a 4 N hydrogen chloride-dioxane solution was added, the solvent was removed under reduced pressure and then the resulting residue was crystallized from ethanol-diethyl ether to give 0.46 g of 1-benzyl-4-piperidyl N-(2-biphenylyl)carbamate hydrochloride as colorless crystals.

Melting point: 183 - 184°C (EtOH-$Et_2O$)

| Elemental analysis (for $C_{25}H_{27}N_2O_2Cl$): | | | | |
|---|---|---|---|---|
| | C (%) | H (%) | N (%) | Cl (%) |
| Calcd. | 70.99 | 6.43 | 6.62 | 8.38 |
| Found | 70.86 | 6.46 | 6.59 | 8.49 |

The following compound was prepared in the same manner as described in Example 11.

Example 12

1-Benzyl-4-piperidyl N-(2-benzylphenyl)carbamate monohydrochloride

Starting compound: 2-benzylbenzoic acid

Melting point: 218 - 222°C (EtOH-$CH_3CN$)

| Elemental analysis (for $C_{26}H_{29}N_2O_2Cl$): | | | | |
|---|---|---|---|---|
| | C (%) | H (%) | N (%) | Cl (%) |
| Calcd. | 71.46 | 6.69 | 6.41 | 8.11 |
| Found | 71.43 | 6.70 | 6.47 | 8.07 |

Example 13

A 9.2 ml portion of 1-chloroethyl chloroformate was added dropwise at room temperature to 70 ml of a 1,2-dichloroethane solution containing 11.0 g of 1-benzyl-4-piperidyl N-(2-biphenylyl)carbamate, and the resulting mixture was stirred under reflux for 3 hours. The reaction solution was cooled to room temperature and the solvent was removed under reduced pressure. The resulting residue was mixed with 70 ml of methanol and stirred overnight under reflux. The solvent was removed under reduced pressure and the resulting residue was mixed with 200 ml of a saturated sodium bicarbonate aqueous solution, followed by extraction with chloroform. The resulting organic layer was washed with brine and dried over anhydrous magnesium sulfate and then the solvent was removed under reduced pressure. The resulting residue was solidified with acetonitrile-diethyl ether to give 5.96 g of 4-piperidyl N-(2-biphenylyl)carbamate as colorless solid. A 0.76 g portion thereof was recrystallized from acetonitrile to give 0.40 g of 4-piperidyl N-(2-biphenylyl)carbamate as colorless crystals.

Melting point: 147 - 149°C ($CH_3CN$)

| Elemental analysis (for $C_{18}H_{20}N_2O_2$): | | | |
|---|---|---|---|
| | C (%) | H (%) | N (%) |
| Calcd. | 72.95 | 6.80 | 9.45 |
| Found | 72.91 | 6.75 | 9.49 |

Example 14

A 0.72 g portion of sodium triacetoxyborohydride was added in small portions to 15 ml of a 1,2-dichloroethane solution containing 0.50 g of 4-piperidyl N-(2-biphenylyl)carbamate and 0.20 ml of 4-methylbenzaldehyde, and the resulting mixture was stirred at room temperature for 3 days. The reaction mixture was mixed with brine, adjusted to pH 9 with saturated sodium bicarbonate aqueous solution, followed by extraction with chloroform. The organic layer was washed with brine and dried over anhydrous sodium sulfate and then the solvent was removed under reduced pressure. The resulting residue was purified by silica gel column chromatography (chloroform/methanol = 50/1) to give 0.60 g of 1-(4-methylbenzyl)-4-piperidyl N-(2-biphenylyl)carbamate as colorless oil. This was dissolved in methanol and mixed with 0.14 g of oxalic acid, the solvent was removed under reduced pressure and then solid product obtained by solidification with ethanol-diethyl ether was recrystallized from acetonitrile-diisopropyl ether to give 0.45 g of 1-(4-methylbenzyl)-4-piperidyl N-(2-biphenylyl)carbamate monooxalate as colorless crystals.

Melting point: 103 - 107°C ($CH_3CN$-i-$Pr_2O$)

| Elemental analysis (for $C_{28}H_{30}N_2O_6 \cdot 0.2H_2O$): | | | |
|---|---|---|---|
| | C (%) | H (%) | N (%) |
| Calcd. | 68.06 | 6.20 | 5.67 |
| Found | 68.36 | 6.59 | 5.33 |

The following compounds of Examples 15 to 21 were prepared by the procedure similar to that of Example 14.

Example 15

1-(3-Hydroxybenzyl)-4-piperidyl N-(2-biphenylyl)carbamate monooxalate Starting compound: 3-hydroxybenzaldehyde

Melting point: 167 - 170°C (EtOH-$CH_3CN$)

| Elemental analysis (for $C_{27}H_{28}N_2O_7$): | | | |
|---|---|---|---|
| | C (%) | H (%) | N (%) |
| Calcd. | 65.84 | 5.73 | 5.69 |
| Found | 65.79 | 5.68 | 5.73 |

Example 16

1-(4-Hydroxybenzyl)-4-piperidyl N-(2-biphenylyl)carbamate hemioxalate

Starting compound: 4-hydroxybenzaldehyde

Melting point: 140 - 142°C (EtOH-$H_2O$)

| Elemental analysis (for $C_{26}H_{27}N_2O_5 \cdot 1.75H_2O$): | | | |
|---|---|---|---|
| | C (%) | H (%) | N (%) |
| Calcd. | 65.19 | 6.42 | 5.85 |
| Found | 65.18 | 6.28 | 5.84 |

Example 17

1-(3-Methoxybenzyl)-4-piperidyl N-(2-biphenylyl)carbamate monooxalate

Starting compound: m-anisaldehyde

Melting point: 136 - 138°C ($EtOH-Et_2O$)

| Elemental analysis (for $C_{28}H_{30}N_2O_7$): | | | |
|---|---|---|---|
| | C (%) | H (%) | N (%) |
| Calcd. | 66.39 | 5.97 | 5.53 |
| Found | 66.25 | 5.94 | 5.47 |

Example 18

1-(4-Methoxybenzyl)-4-piperidyl N-(2-biphenylyl)carbamate monooxalate

Starting compound: p-anisaldehyde

Melting point: 131 - 134°C ($CH_3CN$-i-$Pr_2O$)

| Elemental analysis (for $C_{28}H_{30}N_2O_7$): | | | |
|---|---|---|---|
| | C (%) | H (%) | N (%) |
| Calcd. | 66.39 | 5.97 | 5.53 |
| Found | 66.06 | 5.92 | 5.48 |

Example 19

1-(4-Methylthiobenzyl)-4-piperidyl N-(2-biphenylyl)carbamate monooxalate

Starting compound: 4-(methylthio)benzaldehyde

Melting point: 149 - 150°C ($EtOH-Et_2O$)

| Elemental analysis (for $C_{28}H_{30}N_2O_6S$): | | | | |
|---|---|---|---|---|
| | C (%) | H (%) | N (%) | S (%) |
| Calcd. | 64.35 | 5.79 | 5.36 | 6.14 |
| Found | 64.43 | 5.75 | 5.35 | 6.22 |

Example 20

1-(3,4-Methylenedioxybenzyl)-4-piperidyl N-(2-biphenylyl)carbamate monofumarate

Starting compound: piperonal

Melting point: 161 - 162°C ($EtOH-Et_2O$)

| Elemental analysis (for $C_{30}H_{30}N_2O_8$): | | | |
|---|---|---|---|
| | C (%) | H (%) | N (%) |
| Calcd. | 65.92 | 5.53 | 5.13 |
| Found | 65.63 | 5.52 | 5.08 |

Example 21

1-(4-Dimethylaminobenzyl)-4-piperidyl N-(2-biphenylyl)carbamate dioxalate

Starting compound: 4-(dimethylamino)benzaldehyde

Melting point: 149 - 152°C ($CH_3CN$-i-$Pr_2O$)

| Elemental analysis (for $C_{31}H_{35}N_3O_{10}$): | | | |
|---|---|---|---|
| | C (%) | H (%) | N (%) |
| Calcd. | 61.08 | 5.79 | 6.89 |
| Found | 60.97 | 5.76 | 6.86 |

Example 22

A 0.93 g portion of potassium carbonate and 0.58 g of 3-nitrobenzyl chloride were added at room temperature to 15 ml of an acetonitrile solution containing 1.0 g of 4-piperidyl N-(2-biphenylyl)carbamate, and the reaction mixture was stirred at room temperature for 30 hours. Insoluble matter was removed by filtration, the resulting filtrate was concentrated under reduced pressure and then the resulting residue was purified by silica gel column chromatography (chloroform/methanol = 98/2) to give 1.5 g of 1-(3-nitrobenzyl)-4-piperidyl N-(2-biphenylyl)carbamate in a yellow amorphous form. A 0.62 g portion thereof was recrystallized from ethanol to give 0.20 g of 1-(3-nitrobenzyl)-4-piperidyl N-(2-biphenylyl)carbamate as colorless crystals.

Melting point: 93 - 94°C (EtOH)

| Elemental analysis (for $C_{25}H_{25}N_3O_4$): | | | |
|---|---|---|---|
| | C (%) | H (%) | N (%) |
| Calcd. | 69.59 | 5.84 | 9.74 |
| Found | 69.63 | 5.82 | 9.68 |

The following compounds of Examples 23 to 32 were prepared in the same manner as described Example 22.

Example 23

1-(4-Nitrobenzyl)-4-piperidyl N-(2-biphenylyl)carbamate

Starting compound: 4-nitrobenzyl bromide

Melting point: 117 - 118°C (EtOH)

| Elemental analysis (for $C_{25}H_{25}N_3O_4$): | | | |
|---|---|---|---|
| | C (%) | H (%) | N (%) |
| Calcd. | 69.59 | 5.84 | 9.74 |
| Found | 69.46 | 5.82 | 9.70 |

Example 24

1-(3-Bromobenzyl)-4-piperidyl N-(2-biphenylyl)carbamate monooxalate

Starting compound: 3-bromobenzyl bromide

Melting point: 158 - 160°C (MeOH-i-$Pr_2O$)

| Elemental analysis (for $C_{27}H_{27}N_2O_6Br$): | | | | |
|---|---|---|---|---|
| | C (%) | H (%) | N (%) | Br (%) |
| Calcd. | 58.39 | 4.90 | 5.04 | 14.39 |
| Found | 58.33 | 4.86 | 5.03 | 14.13 |

Example 25

1-(3-Cyanobenzyl)-4-piperidyl N-(2-biphenylyl)carbamate monooxalate

Starting compound: 3-bromomethylbenzonitrile

Melting point: 180 - 184°C (MeOH-EtOH)

| Elemental analysis (for $C_{28}H_{27}N_3O_6$): | | | |
|---|---|---|---|
| | C (%) | H (%) | N (%) |
| Calcd. | 67.06 | 5.43 | 8.38 |
| Found | 66.90 | 5.39 | 8.41 |

Example 26

1-(3-Fluorobenzyl)-4-piperidyl N-(2-biphenylyl)carbamate monooxalate

Starting compound: 3-fluorobenzyl bromide

Melting point: 185 - 186°C (EtOH-$Et_2O$)

| Elemental analysis (for $C_{27}H_{27}N_2O_6F$): | | | | |
|---|---|---|---|---|
| | C (%) | H (%) | N (%) | F (%) |
| Calcd. | 65.58 | 5.50 | 5.66 | 3.84 |
| Found | 65.54 | 5.54 | 5.68 | 3.82 |

Example 27

1-(2-Chlorobenzyl)-4-piperidyl N-(2-biphenylyl)carbamate monooxalate

Starting compound: 2-chlorobenzyl chloride

Melting point: 197 - 198°C (EtOH-$H_2O$)

| Elemental analysis (for $C_{27}H_{27}N_2O_6Cl$): | | | | |
|---|---|---|---|---|
| | C (%) | H (%) | N (%) | Cl (%) |
| Calcd. | 63.47 | 5.33 | 5.48 | 6.94 |
| Found | 63.55 | 5.31 | 5.51 | 6.88 |

Example 28

1-(3-Chlorobenzyl)-4-piperidyl N-(2-biphenylyl)carbamate monooxalate

Starting compound: 3-chlorobenzyl bromide

Melting point: 158 - 159°C (EtOH-$Et_2O$)

| Elemental analysis (for $C_{27}H_{27}N_2O_6Cl$): | | | | |
|---|---|---|---|---|
| | C (%) | H (%) | N (%) | Cl (%) |
| Calcd. | 63.47 | 5.33 | 5.48 | 6.94 |
| Found | 63.60 | 5.36 | 5.45 | 7.17 |

Example 29

1-(2-Methylbenzyl)-4-piperidyl N-(2-biphenylyl)carbamate monooxalate

Starting compound: 2-methylbenzyl bromide

Melting point: 185 - 186°C (EtOH-$Et_2O$)

| Elemental analysis (for $C_{28}H_{30}N_2O_6$): | | | |
|---|---|---|---|
| | C (%) | H (%) | N (%) |
| Calcd. | 68.56 | 6.16 | 5.71 |
| Found | 68.43 | 6.26 | 5.72 |

Example 30

1-(3-Methylbenzyl)-4-piperidyl N-(2-biphenylyl)carbamate monooxalate

Starting compound: 3-methylbenzyl chloride

Melting point: 180 - 181°C (EtOH-$Et_2O$)

| Elemental analysis (for $C_{28}H_{30}N_2O_6$): | | | |
|---|---|---|---|
| | C (%) | H (%) | N (%) |
| Calcd. | 68.56 | 6.16 | 5.71 |
| Found | 68.52 | 6.16 | 5.68 |

Example 31

1-(3-Trifluoromethylbenzyl)-4-piperidyl N-(2-biphenylyl)carbamate monooxalate

Starting compound: 3-trifluoromethylbenzyl bromide

Melting point: 150 - 151°C (EtOH)

| Elemental analysis (for $C_{28}H_{27}N_2O_6F_3$): | | | | |
|---|---|---|---|---|
| | C (%) | H (%) | N (%) | F (%) |
| Calcd. | 61.76 | 5.00 | 5.14 | 10.47 |
| Found | 61.76 | 5.03 | 5.12 | 10.76 |

Example 32

1-(3-Methoxycarbonylbenzyl)-4-piperidyl N-(2-biphenylyl)carbamate monooxalate

Starting compound: methyl 3-bromomethylbenzoate

Melting point: 115 - 116°C (EtOH-$Et_2O$)

| Elemental analysis (for $C_{29}H_{30}N_2O_8 \cdot 0.25H_2O$): | | | |
|---|---|---|---|
| | C (%) | H (%) | N (%) |
| Calcd. | 64.61 | 5.70 | 5.20 |
| Found | 64.66 | 5.59 | 5.21 |

Example 33

A 1.1 g portion of 1-(3-nitrobenzyl)-4-piperidyl N-(2-biphenylyl)carbamate was dissolved in 50 ml of methanol to carry out catalytic reduction in the presence of Raney nickel under an atmosphere of hydrogen. After removal of Raney nickel by filtration, the solvent was removed and the resulting residue was purified by silica gel column chromatography (chloroform/methanol = 50/1) to give 0.68 g of 1-(3-aminobenzyl)-4-piperidyl N-(2-biphenylyl)carbamate in a pale yellow amorphous form. This was dissolved in 10 ml of methanol and mixed with 2 ml of 4 N hydrogen chloride-ethyl acetate solution, the solvent was removed under reduced pressure and then the resulting pale yellow solid was recrystallized from ethanol-diisopropyl ether to give 0.15 g of 1-(3-aminobenzyl)-4-piperidyl N-(2-biphenylyl)carbamate dihydrochloride as colorless crystals.

Melting point: 189 - 193°C (EtOH-i-$Pr_2O$)

| Elemental analysis (for $C_{25}H_{29}N_3O_2Cl_2 \cdot H_2O$): | | | | |
|---|---|---|---|---|
| | C (%) | H (%) | N (%) | Cl (%) |
| Calcd. | 60.98 | 6.35 | 8.53 | 14.40 |
| Found | 61.25 | 6.16 | 8.32 | 14.06 |

The following compound was prepared in the same manner as described in Example 33.

Example 34

1-(4-Aminobenzyl)-4-piperidyl N-(2-biphenylyl)carbamate dioxalate

Starting compound: 1-(4-nitrobenzyl)-4-piperidyl N-(2-biphenylyl)carbamate

Melting point: 111 - 114°C ($CH_3CN$-i-$Pr_2O$)

| Elemental analysis (for $C_{29}H_{31}N_3O_{10} \cdot 0.25H_2O$): | | | |
|---|---|---|---|
| | C (%) | H (%) | N (%) |
| Calcd. | 59.43 | 5.42 | 7.17 |
| Found | 59.43 | 5.34 | 7.22 |

Example 35

A 0.23 ml portion of methanesulfonyl chloride was added dropwise to 12 ml of an ice-cooled dichloromethane solution containing 0.64 g of N-[4-(hydroxymethyl)benzyl]trifluoroacetamide and 0.33 g of triethylamine, and the reaction mixture was stirred at room temperature for 3 hours. The reaction mixture was poured into water and extracted with dichloromethane and the resulting organic layer was washed with water and brine, and then dried over anhydrous

sodium sulfate, and then the solvent was removed under reduced pressure. The resulting residue was dissolved in 5 ml of dimethylformamide and added dropwise to a mixture of 0.74 g of 4-piperidyl N-(2-biphenylyl)carbamate and 0.37 g of potassium carbonate suspended in 20 ml of ice-cooled dimethylformamide, and the reaction mixture was stirred at room temperature overnight. The reaction solution was poured into water and extracted with ethyl acetate, the resulting organic layer was washed with water and brine in that order and dried over anhydrous sodium sulfate and then the solvent was removed under reduced pressure. The resulting residue was purified by silica gel column chromatography (chloroform/methanol = 100/1) to give 1.19 g of 1-(4-trifluoroacetamidobenzyl)-4-piperidyl N-(2-biphenylyl)carbamate as pale yellow oil. This was dissolved in 20 ml of methanol, mixed with 4 ml of water and 0.20 g of potassium carbonate, followed by overnight stirring at room temperature. The solvent was removed under reduced pressure and the resulting residue was mixed with water, followed by extraction with chloroform-2-propanol. The organic layer was washed with brine and dried over anhydrous sodium sulfate and then the solvent was removed under reduced pressure. The resulting residue was dissolved in 20 ml of ethanol and mixed with 1.5 ml of a 4 N hydrogen chloride-dioxane solution and then the solvent was removed under reduced pressure. Thereafter, the resulting residue was crystallized from ethanol-diethyl ether and then recrystallized from ethanol-diethyl ether to give 0.47 g of 1-(4-aminomethylbenzyl)-4-piperidyl N-(2-biphenylyl)carbamate dihydrochloride as colorless crystals.

Melting point: 225 - 229°C (EtOH-$Et_2O$)

| Elemental analysis (for $C_{26}H_{31}N_3O_2Cl_2 \cdot 0.75H_2O$): | | | | |
|---|---|---|---|---|
| | C (%) | H (%) | N (%) | Cl (%) |
| Calcd. | 62.21 | 6.53 | 8.37 | 14.13 |
| Found | 62.57 | 6.30 | 8.36 | 13.84 |

The following compound was prepared in the same manner as described in Example 3.

Example 36

4-[[N-(2-Biphenylyl)carbamoyl]oxy]-1-ethylquinuclidinium iodide

Starting compound: ethyl iodide

Melting point: 243 - 245°C (decomposition) (2-butanone)

| Elemental analysis (for $C_{22}H_{27}N_2O_2I$): | | | | |
|---|---|---|---|---|
| | C (%) | H (%) | N (%) | Cl (%) |
| Calcd. | 55.24 | 5.69 | 5.86 | 26.53 |
| Found | 55.08 | 5.61 | 5.82 | 26.80 |

The following compounds of Examples 37 to 40 were prepared in the same manner as described in Example 22.

Example 37

1-Cyclohexylmethyl-4-piperidyl N-(2-biphenylyl)carbamate monooxalate

Starting compound: cyclohexylmethyl bromide

Melting point: 180 - 181°C (EtOH-$Et_2O$)

| Elemental analysis (for $C_{27}H_{34}N_2O_6$): | | | |
|---|---|---|---|
| | C (%) | H (%) | N (%) |
| Calcd. | 67.20 | 7.10 | 5.80 |
| Found | 66.99 | 6.97 | 5.82 |

Example 38

1-Phenethyl-4-piperidyl N-(2-biphenylyl)carbamate monohydrochloride

Starting compound: phenethyl bromide

Melting point: 137 - 139°C (EtOH-$Et_2O$)

| Elemental analysis (for $C_{26}H_{29}N_2O_2Cl \cdot 0.5H_2O$): | | | | |
|---|---|---|---|---|
| | C (%) | H (%) | N (%) | Cl (%) |
| Calcd. | 70.02 | 6.78 | 6.28 | 7.95 |
| Found | 70.34 | 7.00 | 6.08 | 7.72 |

Example 39

A mixture of 1-[(1-trityl-1H-benzimidazol-5-yl)methyl]-4-piperidyl N-(2-biphenylyl)carbamate and 1-[(1-trityl-1H-benzimidazol-6-yl)methyl]-4-piperidyl N-(2-biphenylyl)carbamate

Starting compound: A mixture of 5-chloromethyl-1-trityl-1H-benzimidazole and 6-chloromethyl-1-trityl-1H-benzimidazole

Mass spectrum data (m/z, FAB): 669 ($M^+$ + 1)
Nuclear magnetic resonance spectrum (DMSO-$d_6$, TMS internal standard)
δ: 1.15 - 1.30 (1 H, m), 1.35 - 1.50 (0.5 H, m), 1.50 - 1.60 (1 H, m), 1.65 - 1.85 (1.5 H, m), 2.05 - 2.30 (1.5 H, m), 2.50 - 2.65 (0.5 H, m), 3.20 - 3.45 (2 H, m), 4.30 - 4.50 (1 H, m), 6.35 - 6.40 (1 H, m), 6.80 - 7.25 (7 H, m), 7.25 - 7.65 (19 H, m), 7.80 (0.5 H, s), 7.86 (0.5 H, s), 8.61 (1 H, d, J = 8.8 Hz)

Example 40

1-(2-Pyridylmethyl)-4-piperidyl N-(2-biphenylyl)carbamate dihydrochloride

Starting compound: 2-chloromethylpyridine hydrochloride

Melting point: 144 - 150°C (EtOH-$CH_3CN$)

| Elemental analysis (for $C_{24}H_{27}N_3O_2Cl_2 \cdot H_2O$): | | | | |
|---|---|---|---|---|
| | C (%) | H (%) | N (%) | Cl (%) |
| Calcd. | 60.25 | 6.11 | 8.78 | 14.82 |
| Found | 60.51 | 5.85 | 8.81 | 14.75 |

The following compounds of Examples 41 to 45 were prepared in the same manner as described in Example 14.

Example 41

1-Furfuryl-4-piperidyl N-(2-biphenylyl)carbamate oxalate

Starting compound: 2-furaldehyde

Melting point: 163 - 164°C (EtOH-AcOEt)

| Elemental analysis (for $C_{25}H_{26}N_2O_7$): | | | |
|---|---|---|---|
| | C (%) | H (%) | N (%) |
| Calcd. | 64.37 | 5.62 | 6.01 |
| Found | 64.28 | 5.61 | 5.96 |

Example 42

1-(4-Chlorobenzyl)-4-piperidyl N-(2-biphenylyl)carbamate monooxalate

Starting compound: 4-chlorobenzaldehyde

Melting point: 172 - 173°C (EtOH)

| Elemental analysis (for $C_{27}H_{27}N_2O_6Cl \cdot 0.25H_2O$): | | | | |
|---|---|---|---|---|
| | C (%) | H (%) | N (%) | Cl (%) |
| Calcd. | 62.91 | 5.38 | 5.43 | 6.88 |
| Found | 62.88 | 5.21 | 5.42 | 7.00 |

Example 43

1-(3-Furylmethyl)-4-piperidyl N-(2-biphenylyl)carbamate monooxalate

Starting compound: 3-furaldehyde

Melting point: 165 - 166°C (EtOH)

| Elemental analysis (for $C_{25}H_{26}N_2O_7$): | | | |
|---|---|---|---|
| | C (%) | H (%) | N (%) |
| Calcd. | 64.37 | 5.62 | 6.01 |
| Found | 64.15 | 5.68 | 5.88 |

Example 44

1-(2-Thenyl)-4-piperidyl N-(2-biphenylyl)carbamate monooxalate

Starting compound: 2-thiophenecarbaldehyde

Melting point: 103 - 105°C (EtOH-$Et_2O$)

| Elemental analysis (for $C_{25}H_{26}N_2O_6S \cdot 0.25H_2O$): | | | | |
|---|---|---|---|---|
| | C (%) | H (%) | N (%) | S (%) |
| Calcd. | 61.65 | 5.48 | 5.75 | 6.58 |
| Found | 61.69 | 5.52 | 5.59 | 6.56 |

Example 45

1-(3-Thenyl)-4-piperidyl N-(2-biphenylyl)carbamate monooxalate

Starting compound: 3-thiophenecarbaldehyde

Melting point: 147 - 149°C (AcOEt-$CH_3CN$)

| Elemental analysis (for $C_{25}H_{26}N_2O_6S$): | | | | |
|---|---|---|---|---|
| | C (%) | H (%) | N (%) | S (%) |
| Calcd. | 62.23 | 5.43 | 5.81 | 6.65 |
| Found | 62.20 | 5.42 | 5.77 | 6.65 |

The following compounds of Examples 46 to 48 were prepared in the same manner as described in Example 1.

Example 46

3-Quinuclidinyl N-(2-benzoylphenyl)carbamate monohydrochloride

Starting compound: 2-benzoylbenzoic acid

Melting point: 236 - 238°C (decomposition) (EtOH)

| Elemental analysis (for $C_{21}H_{23}N_2O_3Cl \cdot 0.1H_2O$): | | | | |
|---|---|---|---|---|
| | C (%) | H (%) | N (%) | Cl (%) |
| Calcd. | 64.89 | 6.02 | 7.21 | 9.12 |
| Found | 64.84 | 5.97 | 7.19 | 8.97 |

Example 47

3-Quinuclidinyl N-(2'-methyl-2-biphenylyl)carbamate monohydrochloride

Starting compound: 2'-methyl-2-biphenylcarboxylic acid

Melting point: 195 - 196°C ($EtOH-Et_2O$)

| Elemental analysis (for $C_{21}H_{25}N_2O_2Cl$): | | | | |
|---|---|---|---|---|
| | C (%) | H (%) | N (%) | Cl (%) |
| Calcd. | 67.64 | 6.76 | 7.51 | 9.51 |
| Found | 67.41 | 6.75 | 7.50 | 9.39 |

Example 48

3-Quinuclidinyl N-(2'-nitro-2-biphenylyl)carbamate monofumarate

Starting compound: 2'-nitro-2-biphenylcarboxylic acid

Melting point: 156 - 157°C ($CH_3CN-Et_2O$)

| Elemental analysis (for $C_{24}H_{25}N_3O_8$): | | | |
|---|---|---|---|
| | C (%) | H (%) | N (%) |
| Calcd. | 57.77 | 5.07 | 9.19 |
| Found | 57.78 | 5.13 | 9.32 |

Example 49

To a 30 ml of acetone solution containing 0.97 g portion of the mixture of 1-[(1-trityl-1H-benzimidazol-5-yl)methyl]-4-piperidyl N-(2-biphenylyl)carbamate and 1-[(1-trityl-1H-benzimidazol-6-yl)methyl]-4-piperidyl N-(2-biphenylyl)carbamate was added 15 ml of 1 N hydrochloric acid, and the reaction mixture was stirred at room temperature for 18 hours. The reaction mixture was concentrated under reduced pressure, adjusted to pH 9 by the addition of a saturated sodium bicarbonate aqueous solution and then extracted with chloroform. The resulting organic layer was washed with brine and dried over anhydrous sodium sulfate. Then, the solvent was removed under reduced pressure to give 0.71 g of 1-(1H-benzimidazol-5-ylmethyl)-4-piperidyl N-(2-biphenylyl)carbamate as colorless oil. A 0.55 g portion thereof was dissolved in 20 ml of ethanol and mixed with 0.11 g of oxalic acid and then the solvent was removed under reduced pressure. The resulting residue was purified by silica gel column chromatography (chloroform/methanol = 10/1 → 5/1) to give 0.20 g of 1-(1H-benzimidazol-5-ylmethyl)-4-piperidyl N-(2-biphenylyl)carbamate 1.5 oxalate as colorless solid which was subsequently recrystallized from ethanol-diethyl ether to give 0.10 g of 1-(1H-benzimidazol-5-ylmethyl)-4-piperidyl N-(2-biphenylyl)carbamate sesqioxalate as colorless crystals.

Melting point: 191 - 193°C ($EtOH-Et_2O$)

| Elemental analysis (for $C_{29}H_{29}N_4O_8 \cdot H_2O$): | | | |
|---|---|---|---|
| | C (%) | H (%) | N (%) |
| Calcd. | 60.10 | 5.39 | 9.67 |
| Found | 60.12 | 5.17 | 9.66 |

Example 50

A 6 ml portion of a dichloromethane solution containing 0.60 g of 3-quinuclidinyl N-(2'-methoxy-2-biphenylyl)carbamate was cooled to -60°C in a stream of argon, 3.7 ml of a 1 M boron tribromide-dichloromethane solution was added dropwise at -50°C or below and then the resulting mixture was stirred for 3 hours during which the temperature was gradually increased to -10°C. The reaction mixture was cooled to -50°C, mixed with 3.0 ml of triethylamine and 1.5 ml of methanol in that order, returned to room temperature, mixed with water and then extracted with dichloromethane. The organic layer was washed with brine and dried over anhydrous sodium sulfate and then the solvent was removed under reduced pressure. The resulting residue was purified by silica gel column chromatography (chloroform/methanol = 30/1 → 10/1) to give 0.25 g of 3-quinuclidinyl N-(2'-hydroxy-2-biphenylyl)carbamate. This was dissolved in 10 ml of ethanol, 63 mg of oxalic acid was added, the solvent was removed under reduced pressure, and then the resulting solid was recrystallized from methanol to give 0.18 g of 3-quinuclidinyl N-(2'-hydroxy-2-biphenylyl)carbamate monooxalate as colorless crystals.

Melting point: 153 - 155°C (MeOH)

| Elemental analysis (for $C_{22}H_{24}N_2O_7 \cdot 0.75H_2O$): | | | |
|---|---|---|---|
| | C (%) | H (%) | N (%) |
| Calcd. | 59.79 | 5.82 | 6.34 |
| Found | 59.64 | 5.94 | 6.36 |

Example 51

A 0.80 g portion of 10% palladium-carbon was added to 150 ml of an ethanol solution containing 7.40 g of 3-quinuclidinyl N-(2'-nitro-2-biphenylyl)carbamate to carry out catalytic hydrogenation in an atmosphere of hydrogen. After removal of the catalyst by filtration, the solvent was removed under reduced pressure and the resulting residue was purified by silica gel column chromatography (chloroform/methanol/28% aqueous ammonia = 10/1/0.1) to give 6.27 g of 3-quinuclidinyl N-(2'-amino-2-biphenylyl)carbamate as pale yellow foam. A 2.53 g portion of this was dissolved in 50 ml of ethanol and mixed with 4 ml of 4 N hydrogen chloride-dioxane solution, the solvent was removed under reduced pressure, the resulting residue was solidified with ethanol-diethyl ether and then 1.69 g of the resulting solid substance was recrystallized twice from ethanol to give 3-quinuclidinyl N-(2'-amino-2-biphenylyl)carbamate 1.9 hydrochloride as colorless crystals.

Melting point: 156 - 158°C (EtOH)

| Elemental analysis (for $C_{20}H_{23}N_3O_2 \cdot 1.9HCl \cdot H_2O$): | | | | |
|---|---|---|---|---|
| | C (%) | H (%) | N (%) | Cl (%) |
| Calcd. | 56.56 | 6.38 | 9.89 | 15.86 |
| Found | 56.19 | 6.61 | 9.89 | 15.67 |

Example 52

A 0.21 g portion of sodium borohydride was added in small portions to 40 ml of an ethanol solution containing 1.91 g of 3-quinuclidinyl N-(2-benzoylphenyl)carbamate under ice-cooling, and the reaction mixture was stirred at room temperature for 3 hours. The solvent was removed under reduced pressure, and the residue was diluted with water and extracted with ethyl acetate. The resulting organic layer was washed with brine and dried over anhydrous sodium sulfate and then the solvent was removed under reduced pressure. The resulting residue was purified by silica gel column chromatography (chloroform/methanol = 20/1 → chloroform/methanol/28% aqueous ammonia = 10/1/0.1) to give 1.08 g of 3-quinuclidinyl N-[2-(α-hydroxybenzyl)phenyl]carbamate as pale yellow foam. This was dissolved in 20 ml of ethanol and mixed with 0.8 ml of a 4 N hydrogen chloride-dioxane solution and then the solvent was removed under reduced pressure. The resulting residue was solidified by ethanol-diethyl ether, followed by recrystallization from ethanol to give 0.34 g of 3-quinuclidinyl N-[2-(α-hydroxybenzyl)phenyl]carbamate monohydrochloride as colorless crystals.

Melting point: 202 - 203°C (EtOH)

| Elemental analysis (for $C_{21}H_{25}N_2O_3Cl$): | | | | |
|---|---|---|---|---|
| | C (%) | H (%) | N (%) | Cl (%) |
| Calcd. | 64.86 | 6.48 | 7.20 | 9.12 |
| Found | 64.76 | 6.53 | 7.22 | 9.12 |

Example 53

A 0.05 g portion of sodium hydride (60%) was added to 30 ml of a toluene solution containing 0.93 g of ethyl N-[2-(2-cyclopenten-1-yl)phenyl]carbamate and 0.76 g of 3-quinuclidinol, and the reaction mixture was stirred overnight at 135°C with removal of formed ethanol. The reaction mixture was cooled to room temperature, mixed with brine and then extracted with ethyl acetate. The resulting organic layer was dried over anhydrous sodium sulfate, the solvent was removed under reduced pressure and then the resulting residue was purified by silica gel column chromatography (chloroform/methanol = 30/1) to give 1.10 g of 3-quinuclidinyl N-[2-(2-cyclopenten-1-yl)phenyl]carbamate as pale yellow oil. This was dissolved in 30 ml of ethanol and mixed with 1.4 ml of a 4 N hydrogen chloride-dioxane solution and then the solvent was removed under reduced pressure. The resulting residue was solidified by adding acetonitrile and ethyl acetate, followed by recrystallization from ethanol-ethyl acetate to give 0.44 g of 3-quinuclidinyl N-[2-(2-cyclopenten-1-yl)phenyl]carbamate hydrochloride as colorless crystals.

Melting point: 181 - 182°C (EtOH-AcOEt)

| Elemental analysis (for $C_{19}H_{25}N_2O_2Cl \cdot 0.25H_2O$): | | | | |
|---|---|---|---|---|
| | C (%) | H (%) | N (%) | Cl (%) |
| Calcd. | 64.58 | 7.27 | 7.93 | 10.03 |
| Found | 64.78 | 7.14 | 7.90 | 10.20 |

Example 54

A 0.2 ml portion of a 4 N hydrogen chloride-dioxane solution and 0.20 g of 10% palladium-carbon were added to 5 ml of an ethanol solution containing 0.23 g of 3-quinuclidinyl N-[2-(2-cyclohexen-1-yl)phenyl]carbamate, followed by catalytic hydrogenation under atmosphere of hydrogen. After removal of catalyst by filtration, the solvent was removed under reduced pressure. The resulting residue was mixed with a sodium bicarbonate aqueous solution and extracted with chloroform. The resulting organic layer was dried over anhydrous sodium sulfate and the solvent was removed under reduced pressure. The resulting residue was dissolved in 5 ml of ethanol, 26 mg of oxalic acid was added, the solvent was removed under reduced pressure, followed by crystallization from acetonitrile-diethyl ether to give 0.08 g of 3-quinuclidinyl N-(2-cyclohexylphenyl)carbamate oxalate as colorless crystals.

Melting point: 102 - 103°C ($CH_3CN$-$Et_2O$)

| Elemental analysis (for $C_{22}H_{30}N_2O_6 \cdot 0.75H_2O$): | | | |
|---|---|---|---|
| | C (%) | H (%) | N (%) |
| Calcd. | 61.17 | 7.35 | 6.48 |
| Found | 61.29 | 7.33 | 6.50 |

Example 55

A 0.64 g portion of 3-quinuclidinyl N-(2-biphenylyl)carbamate was dissolved in 15 ml of dichloromethane and 0.47 g of m-chloroperbenzoic acid was added under ice-cooling, and the reaction mixture was stirred at room temperature for 3.5 days. The reaction solution was mixed with a sodium carbonate aqueous solution the mixture was extracted with chloroform, the resulting organic layer was dried over anhydrous sodium sulfate and then the solvent was removed under reduced pressure to give 0.40 g of 1-oxido-3-quinuclidinyl N-(2-biphenylyl)carbamate as colorless solid which was subsequently recrystallized from ethanol-diethyl ether to give 0.29 g of 1-oxido-3-quinuclidinyl N-(2-biphenylyl)carbamate as colorless crystals.

Melting point: 190 - 191°C
(decomposition) ($EtOH-Et_2O$)

| Elemental analysis (for $C_{20}H_{22}N_2O_3 \cdot 0.2H_2O$): | | | |
|---|---|---|---|
| | C (%) | H (%) | N (%) |
| Calcd. | 70.24 | 6.60 | 8.19 |
| Found | 70.41 | 6.56 | 8.05 |

Example 56

A 1.00 g portion of 3-quinuclidinyl N-(2-phenylthiophenyl)carbamate was dissolved in 30 ml of ethanol, the resulting solution was mixed with 0.80 ml of a 4 N hydrogen chloride-ethyl acetate solution and then the solvent was removed under reduced pressure. The resulting residue was dissolved in 25 ml of ethanol and added dropwise at room temperature to 24 ml of an aqueous solution containing 1.80 g of sodium periodate, and the resulting mixture was stirred at room temperature for 2 days, mixed with 12 ml of an aqueous solution containing 1.20 g of sodium periodate, followed by stirring at room temperature for 2 days. The reaction solution was concentrated under reduced pressure, and the resulting residue was mixed with water and extracted with chloroform. The resulting organic layer was washed with brine and dried over anhydrous sodium sulfate and then the solvent was removed under reduced pressure. The resulting residue was purified by silica gel column chromatography (chloroform/methanol = 20/1) to give 0.56 g of 3-quinuclidinyl N-(2-phenylsulfonylphenyl)carbamate. This was dissolved in 10 ml of ethanol and mixed with 0.4 ml of 4 N hydrogen chloride-dioxane solution, and the solvent was removed under reduced pressure. The resulting residue was solidified by adding ethyl acetate and then recrystallized from ethanol-ethyl acetate to give 0.35 g of 3-quinuclidinyl N-(2-phenylsulfonylphenyl)carbamate hydrochloride as colorless crystals.

Melting point: 161 - 162°C (EtOH-AcOEt)

| Elemental analysis (for $C_{20}H_{23}N_2O_4SCl \cdot 0.25H_2O$): | | | | | |
|---|---|---|---|---|---|
| | C (%) | H (%) | N (%) | S (%) | Cl (%) |
| Calcd. | 56.20 | 5.54 | 6.55 | 7.50 | 8.29 |
| Found | 56.18 | 5.43 | 6.52 | 7.39 | 8.23 |

Example 57

To 1.0 g of 3-quinuclidinyl N-(2-phenylthiophenyl)carbamate dissolved in 20 ml of ethanol was added 0.8 ml of 4 N hydrogen chloride-ethyl acetate solution, and then the solvent was removed under reduced pressure. The resulting residue was dissolved in 25 ml of ethanol and added dropwise at room temperature to 8 ml of an aqueous solution containing 0.60 g of sodium periodate, and the reaction mixture was stirred at room temperature overnight. The reaction mixture was concentrated under reduced pressure, and the residue was made into alkaline with saturated sodium bicarbonate aqueous solution, followed by extraction with chloroform. The resulting organic layer was dried over anhydrous sodium sulfate and then the solvent was removed under reduced pressure. The resulting residue was dissolved in 30 ml of methanol, 0.30 g of fumaric acid was added and then the solvent was removed under reduced pressure. The resulting residue was crystallized from methanol-acetonitrile, followed by recrystallization from ethanol to give 0.34 g of 3-quinuclidinyl N-(2-phenylsulfinylphenyl)carbamate monofumarate.

Melting point: 178 - 179°C (EtOH)

| Elemental analysis (for $C_{24}H_{26}N_2O_7S$): | | | | |
|---|---|---|---|---|
| | C (%) | H (%) | N (%) | S (%) |
| Calcd. | 59.25 | 5.39 | 5.76 | 6.59 |
| Found | 59.02 | 5.35 | 5.73 | 6.68 |

The following compound was prepared in the same manner as described in Example 1.

Example 58

3α-8-Methyl-8-azabicyclo[3.2.1]octan-3-yl N-(2-biphenylyl)carbamate monohydrochloride

Starting compound: tropine

Melting point: 262 - 263°C (EtOH-$Et_2O$)

| Elemental analysis (for $C_{21}H_{25}N_2O_2Cl$): | | | | |
|---|---|---|---|---|
| | C (%) | H (%) | N (%) | Cl (%) |
| Calcd. | 67.64 | 6.76 | 7.51 | 9.51 |
| Found | 67.34 | 6.83 | 7.41 | 9.59 |

The following compounds of Examples 59 and 60 were prepared in the same manner as described in Example 53.

Example 59

3-Quinuclidinyl N-(2-phenylthiophenyl)carbamate monohydrochloride

Starting compound: methyl N-(2-phenylthiophenyl)carbamate

Melting point: 231 - 233°C (EtOH-$Et_2O$)

| Elemental analysis (for $C_{20}H_{23}N_2O_2SCl$): | | | | | |
|---|---|---|---|---|---|
| | C (%) | H (%) | N (%) | S (%) | Cl (%) |
| Calcd. | 61.45 | 5.93 | 7.17 | 8.20 | 9.07 |
| Found | 61.32 | 5.90 | 7.14 | 8.25 | 9.33 |

Example 60

3-Quinuclidinyl N-(2-piperidinophenyl)carbamate dioxalate

Starting compound: ethyl N-(2-piperidinophenyl)carbamate

Melting point: 172 - 173°C (EtOH-$Et_2O$)

| Elemental analysis (for $C_{23}H_{31}N_3O_{10} \cdot 0.25H_2O$): | | | |
|---|---|---|---|
| | C (%) | H (%) | N (%) |
| Calcd. | 53.74 | 6.18 | 8.17 |
| Found | 53.72 | 6.15 | 7.97 |

The following compounds of Examples 61 and 62 were prepared in the same manner as described in Example 2.

Example 61

3-Quinuclidinyl N-[2-(2-cyclohexen-1-yl)phenyl]carbamate hemioxalate

Starting compound: 1,3-bis [2-(2-cyclohexen-1-yl)phenyl]urea

Melting point: 98 - 100°C ($CH_3CN$)

| Elemental analysis (for $C_{21}H_{27}N_2O_4 \cdot H_2O$): | | | |
|---|---|---|---|
| | C (%) | H (%) | N (%) |
| Calcd. | 64.76 | 7.51 | 7.19 |
| Found | 64.58 | 7.29 | 7.09 |

Example 62

1-Benzyl-4-piperidyl N-[2-(2-cyclohexen-1-yl)phenyl]carbamate oxalate

Starting compounds: 1,3-bis[2-(2-cyclohexen-1-yl)phenyl]urea and 1-benzyl-4-hydroxypiperidine

Melting point: 118 - 121°C (EtOH-$Et_2O$)

| Elemental analysis (for $C_{27}H_{32}N_2O_6 \cdot 0.25H_2O$): | | | |
|---|---|---|---|
| | C (%) | H (%) | N (%) |
| Calcd. | 66.86 | 6.75 | 5.78 |
| Found | 66.81 | 6.74 | 5.76 |

Example 63

To the solution of 0.30 g portion of 4-quinuclidinyl N-(2-biphenylyl)carbamate dissolved in 3 ml of 2-butanone, 0.14 g of allyl bromide was added, and the reaction mixture was stirred at 60°C for 3 hours. After cooling, the precipitated crystals were collected by filtration and washed with diethyl ether to give 0.40 g of 1-allyl-4-[[N-(2-biphenylyl)carbamoyl]oxy]quinuclidinium bromide as colorless crystals.

Melting point: 181 - 182°C (2-butanone)

| Elemental analysis (for $C_{23}H_{27}N_2O_2Br$): | | | | |
|---|---|---|---|---|
| | C (%) | H (%) | N (%) | Br (%) |
| Calcd. | 62.31 | 6.14 | 6.32 | 18.02 |
| Found | 62.28 | 6.15 | 6.28 | 17.89 |

The following compounds of Examples 64 to 66 were prepared in the same manner as described in Example 3.

Example 64

4-[[N-(2-Biphenylyl)carbamoyl]oxy]-1-isopropylquinuclidinium iodide

Starting compound: isopropyl iodide

Melting point: 236 - 239°C (2-butanone)

| Elemental analysis (for $C_{23}H_{29}N_2O_2I$): | | | | |
|---|---|---|---|---|
| | C (%) | H (%) | N (%) | I (%) |
| Calcd. | 56.10 | 5.94 | 5.69 | 25.77 |
| Found | 56.22 | 6.02 | 5.64 | 25.52 |

Example 65

4-[[N-(2-biphenylyl)carbamoyl]oxy]-1-propylquinuclidinium iodide

Starting compound: propyl iodide

Melting point: 217 - 218°C (decomposition) (2-butanone)

| Elemental analysis (for $C_{23}H_{29}N_2O_2I$): | | | | |
|---|---|---|---|---|
| | C (%) | H (%) | N (%) | I (%) |
| Calcd. | 56.10 | 5.94 | 5.69 | 25.77 |
| Found | 56.06 | 5.92 | 5.70 | 25.84 |

Example 66

1-Benzyl-3-[[N-(2-biphenylyl)carbamoyl]oxy]quinuclidinium bromide

Starting compound: benzyl bromide

Melting point: 208 - 210°C (2-butanone)

| Elemental analysis (for $C_{27}H_{29}N_2O_2Br \cdot 0.25H_2O$): | | | | |
|---|---|---|---|---|
| | C (%) | H (%) | N (%) | Br (%) |
| Calcd. | 65.13 | 5.97 | 5.63 | 16.05 |
| Found | 64.98 | 5.88 | 5.65 | 16.18 |

Structural formulae of the compounds of the present invention prepared in examples 1 to 66 are shown in the following table.

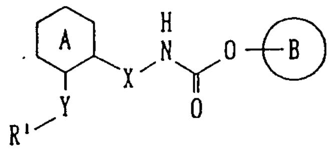

| Example | A | Y | R' | X | B | Salt |
|---|---|---|---|---|---|---|
| 1 | 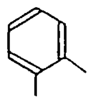 | – | 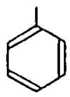 | – | 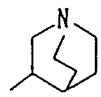 | HCl |
| 2 | 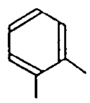 | – | 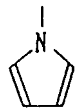 | – | 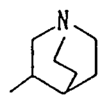 | HCl |
| 3 | 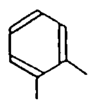 | – | 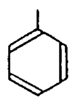 | – | 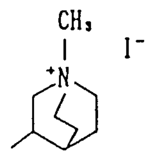 | – |
| 4 | 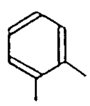 | – | 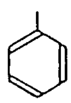 | – | 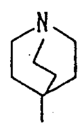 | HCl |
| 5 | 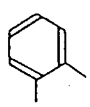 | – | 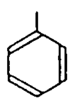 | – | 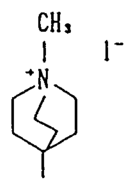 | – |
| 6 | 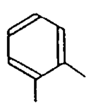 | – | 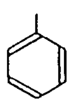 | – | 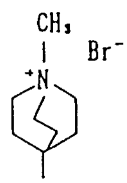 | – |
| 7 | 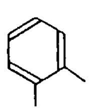 | – | 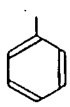 | – | 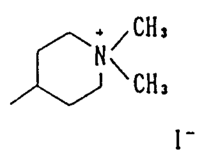 | – |
| 8 | 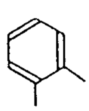 | $-CH_2-$ | 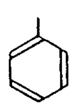 | – | 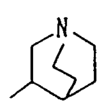 | HBr |

| Example | A | Y | R' | X | B | Salt |
|---|---|---|---|---|---|---|
| 9 | 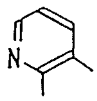 | – | 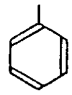 | – | 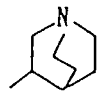 | COOH<br>COOH |
| 10 | 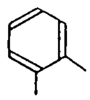 | – | 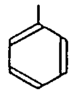 | $-CH_2-$ | 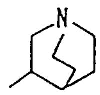 | COOH<br>COOH |
| 11 | 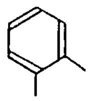 | – | 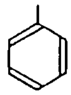 | – | 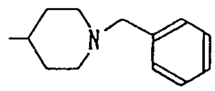 | HCl |
| 12 | 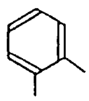 | $-CH_2-$ | 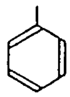 | – | 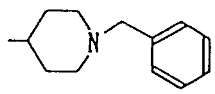 | HCl |
| 13 | 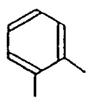 | – | 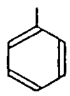 | – | 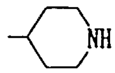 | – |
| 14 | 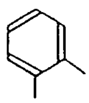 | – | 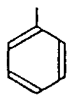 | – | 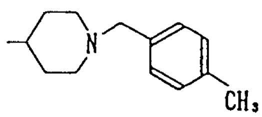 | COOH<br>COOH |
| 15 | 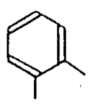 | – | 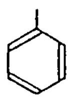 | – | 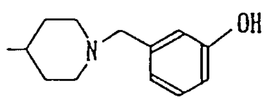 | COOH<br>COOH |
| 16 | 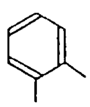 | – | 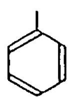 | – | 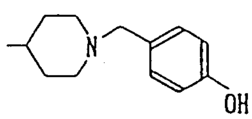 | 0.5 COOH<br>COOH |
| 17 | 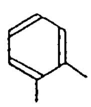 | – | 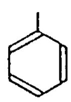 | – | 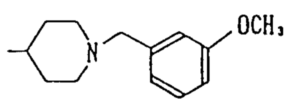 | COOH<br>COOH |
| 18 | 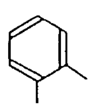 | – | 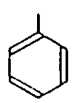 | – | 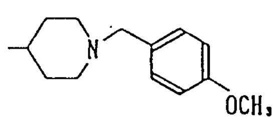 | COOH<br>COOH |
| 19 | 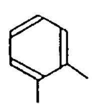 | – | 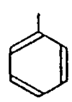 | – | 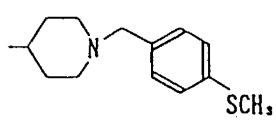 | COOH<br>COOH |

| Example | A | Y | R' | X | B | Salt |
|---|---|---|---|---|---|---|
| 20 | 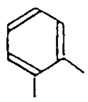 | – | 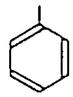 | – | 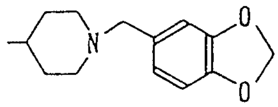 | 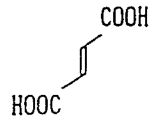 |
| 21 | 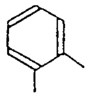 | – | 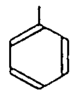 | – | 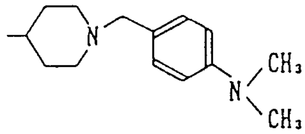 | 2 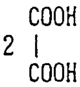 |
| 22 | 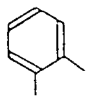 | – | 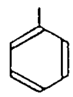 | – | 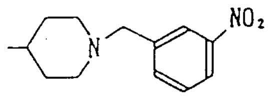 | – |
| 23 | 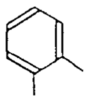 | – | 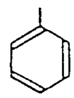 | – | 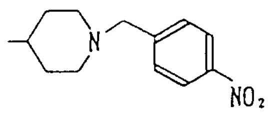 | – |
| 24 | 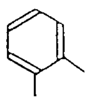 | – | 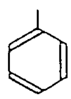 | – | 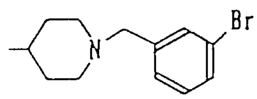 | 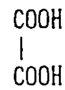 |
| 25 | 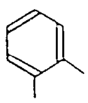 | – | 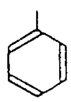 | – | 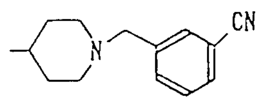 | 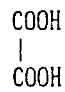 |
| 26 | 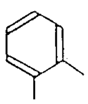 | – | 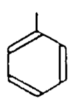 | – | 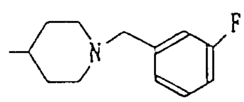 | 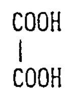 |
| 27 | 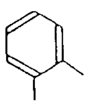 | – | 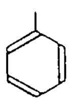 | – | 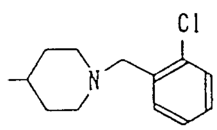 | 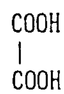 |
| 28 | 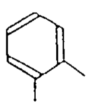 | – | 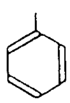 | – | 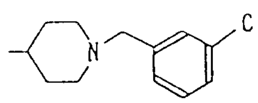 | 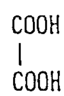 |
| 29 | 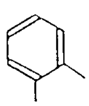 | – | 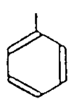 | – | 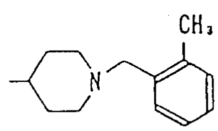 | |
| 30 | 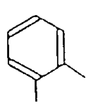 | – | 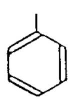 | – | 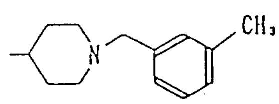 | |

<table>
<tr><th>Example</th><th>A</th><th>Y</th><th>R'</th><th>X</th><th>B</th><th>Salt</th></tr>
<tr><td>31</td><td>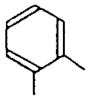</td><td>–</td><td>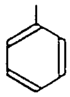</td><td>–</td><td>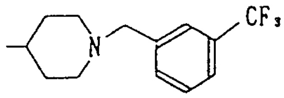</td><td>COOH<br>|<br>COOH</td></tr>
<tr><td>32</td><td>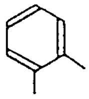</td><td>–</td><td>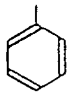</td><td>–</td><td>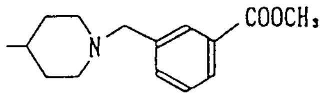</td><td>COOH<br>|<br>COOH</td></tr>
<tr><td>33</td><td>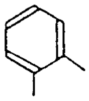</td><td>–</td><td>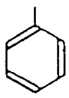</td><td>–</td><td>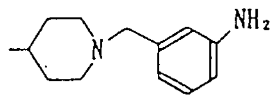</td><td>2HCl</td></tr>
<tr><td>34</td><td>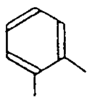</td><td>–</td><td>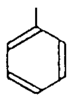</td><td>–</td><td>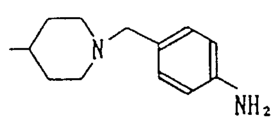</td><td>2 COOH<br>|<br>COOH</td></tr>
<tr><td>35</td><td>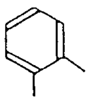</td><td>–</td><td>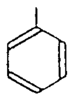</td><td>–</td><td>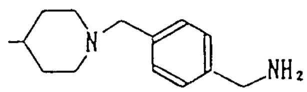</td><td>2HCl</td></tr>
<tr><td>36</td><td>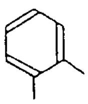</td><td>–</td><td>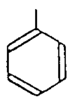</td><td>–</td><td>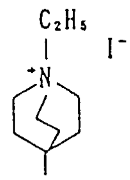</td><td>–</td></tr>
<tr><td>37</td><td>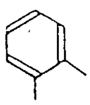</td><td>–</td><td>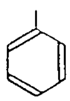</td><td>–</td><td>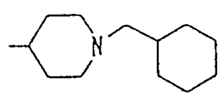</td><td>COOH<br>|<br>COOH</td></tr>
<tr><td>38</td><td>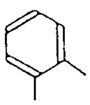</td><td>–</td><td>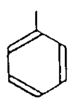</td><td>–</td><td>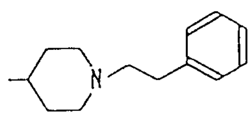</td><td>HCl</td></tr>
<tr><td>39</td><td>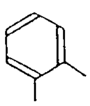</td><td>–</td><td>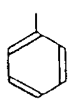</td><td>–</td><td>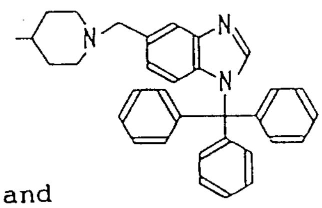<br>and<br>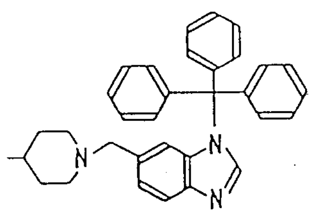</td><td>–</td></tr>
</table>

| Example | A | Y | R' | X | B | Salt |
|---|---|---|---|---|---|---|
| 40 | 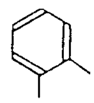 | - | 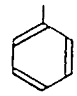 | - | 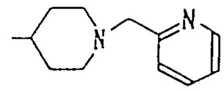 | 2HCl |
| 41 | 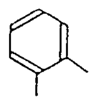 | - | 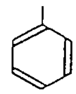 | - | 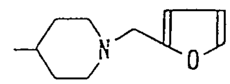 | 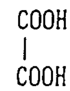 |
| 42 | 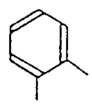 | - | 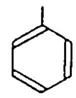 | - | 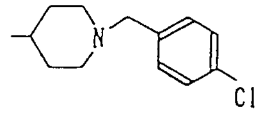 | 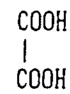 |
| 43 | 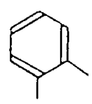 | - | 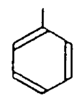 | - | 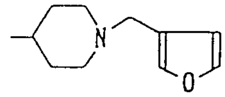 | 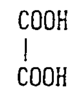 |
| 44 | 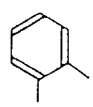 | - | 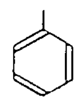 | - | 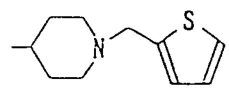 | 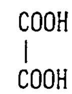 |
| 45 | 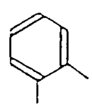 | - | 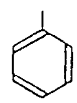 | - | 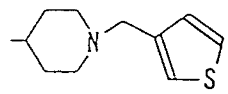 | 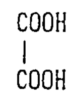 |
| 46 | 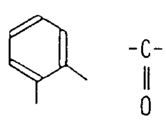 | | 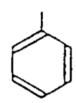 | - | 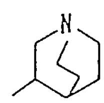 | HCl |
| 47 | 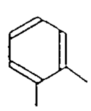 | - | 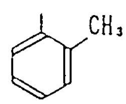 | - | 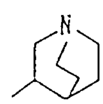 | HCl |
| 48 | 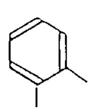 | - | 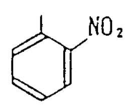 | - | 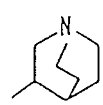 | 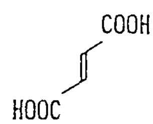 |
| 49 | 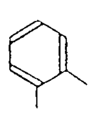 | - | 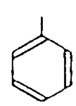 | - | 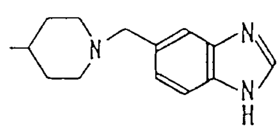 | 1.5 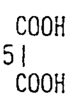 |
| 50 | 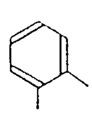 | - | 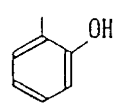 | - | 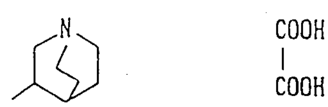 | |

| Example | A | Y | $R^1$ | X | B | Salt |
|---|---|---|---|---|---|---|
| 51 | 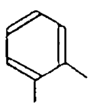 | – | 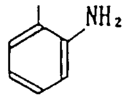 | – | 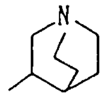 | 1.9HCl |
| 52 | 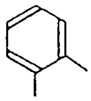 | 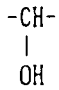 | 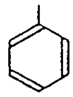 | – | 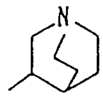 | HCl |
| 53 | 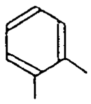 | – | 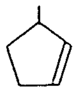 | – | 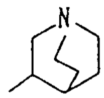 | HCl |
| 54 | 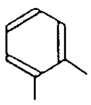 | – | 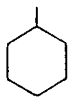 | – | 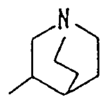 | 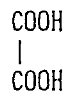 |
| 55 | 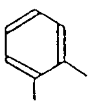 | – | 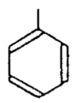 | – | 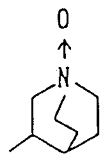 | – |
| 56 | 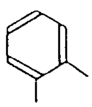 | $-SO_2-$ | 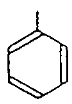 | – | 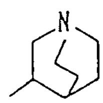 | HCl |
| 57 | 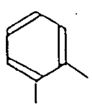 | 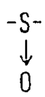 | 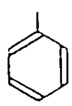 | – | 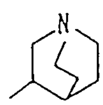 | 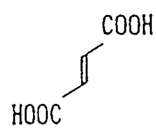 |
| 58 | 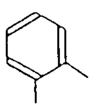 | – | 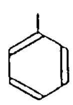 | – | 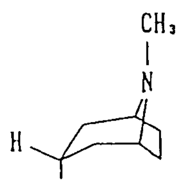 | HCl |
| 59 | 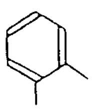 | -S- | 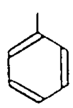 | – | 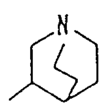 | HCl |
| 60 | 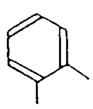 | – | 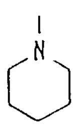 | – | 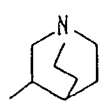 | 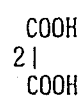 |

| Example | A | Y | R' | X | B | Salt |
|---|---|---|---|---|---|---|
| 61 | 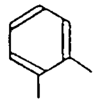 | – | 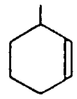 | – | 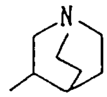 | COOH<br>0.5 \|<br>COOH |
| 62 | 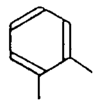 | – | 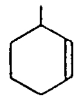 | – | 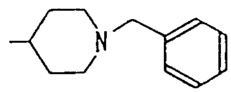 | COOH<br>\|<br>COOH |
| 63 | 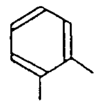 | – | 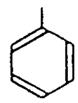 | – | 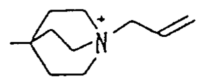 $Br^-$ | – |
| 64 | 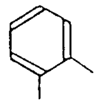 | – | 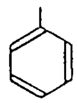 | – | 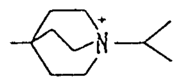 $I^-$ | – |
| 65 | 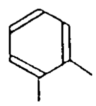 | – | 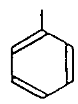 | – | 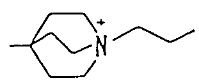 $I^-$ | – |
| 66 | 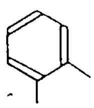 | – | 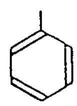 | – | 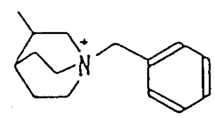 $Br^-$ | – |

In addition to the aforementioned compounds of examples, other compounds of the present invention are shown below. These compounds can be synthesized in accordance with the synthetic pathways and methods described in the aforementioned preparation processes and examples and their modifications known to those skilled in the art, and particular experiments are not required.

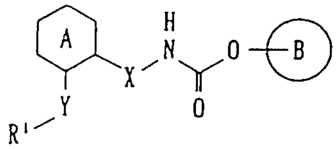

| Compound | A | Y | R' | X | B |
|---|---|---|---|---|---|
| 1 | 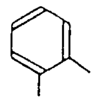 | – | 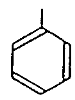 | – | 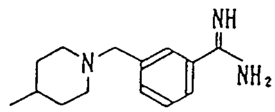 |
| 2 | 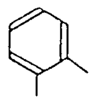 | – | 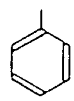 | – | 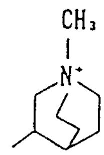 $Br^-$ |
| 3 | 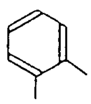 | – | 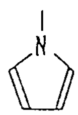 | – | 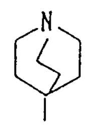 |
| 4 | 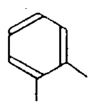 | – | 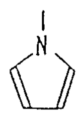 | – | 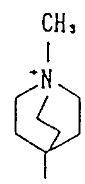 $Br^-$ |
| 5 | 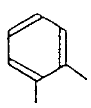 | – | 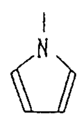 | – | 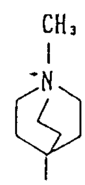 $I^-$ |
| 6 | 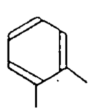 | $-CH_2-$ | 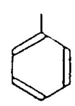 | – | 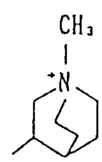 $I^-$ |
| 7 | 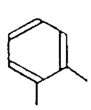 | – | 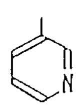 | – | 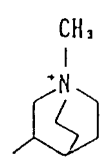 $I^-$ |

| Compound | A | Y | R' | X | B | |
|---|---|---|---|---|---|---|
| 8 | 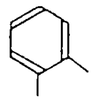 | — | 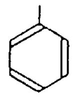 | $-CH_2-$ | 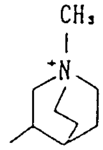 | $I^-$ |
| 9 | 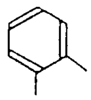 | — | 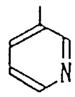 | — | 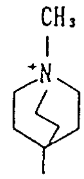 | $I^-$ |
| 10 | 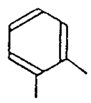 | — | 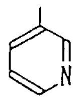 | — | 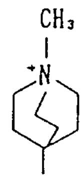 | $Br^-$ |
| 11 | 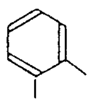 | $-CH_2-$ | 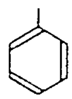 | — | 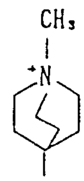 | $Br^-$ |
| 12 | 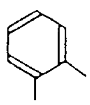 | $-CH_2-$ | 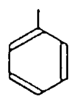 | — | 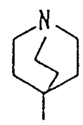 | |
| 13 | 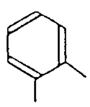 | — | 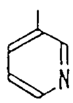 | — | 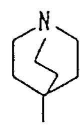 | |
| 14 | 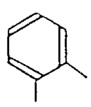 | — | 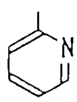 | — | 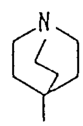 | |
| 15 | 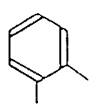 | — | 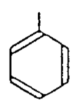 | $-CH_2-$ | 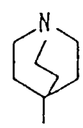 | |
| 16 | 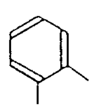 | — | 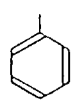 | $-CH_2-$ | 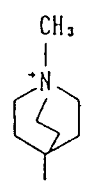 | $I^-$ |

| Compound | A | Y | R¹ | X | B |
|---|---|---|---|---|---|
| 17 | 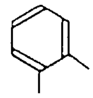 | – | 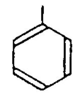 | – | 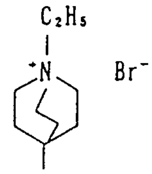 |
| 18 | 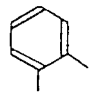 | – | 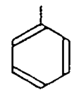 | – | 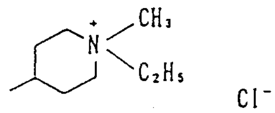 |
| 19 | 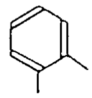 | – | 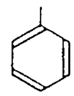 | – | 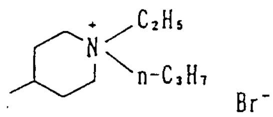 |
| 20 | 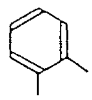 | – | 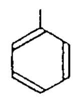 | – | 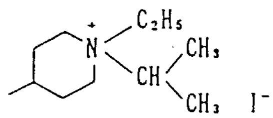 |
| 21 | 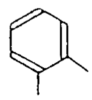 | – | 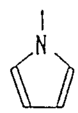 | – | 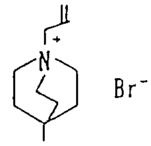 |
| 22 | 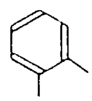 | – | 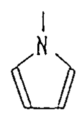 | – | 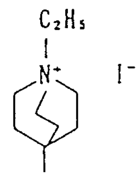 |
| 23 | 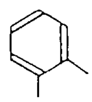 | – | 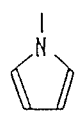 | – | 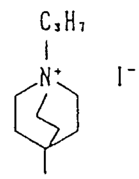 |
| 24 | 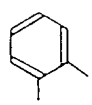 | – | 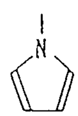 | – | 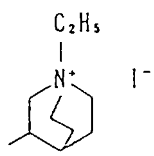 |
| 25 | 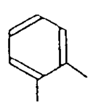 | – | 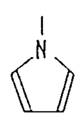 | – | 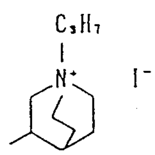 |

| Compound | A | Y | R' | X | B |
|---|---|---|---|---|---|
| 26 | 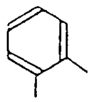 | – | 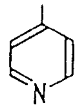 | – | 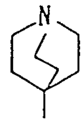 |
| 27 | 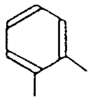 | – | 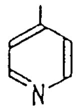 | – | 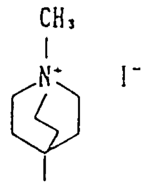 |
| 28 | 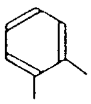 | – | 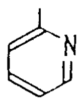 | – | 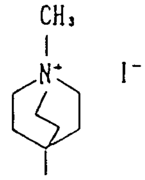 |
| 29 | 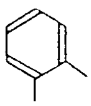 | – | 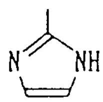 | – | 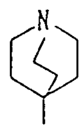 |
| 30 | 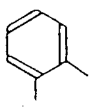 | – | 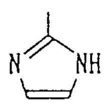 | – | 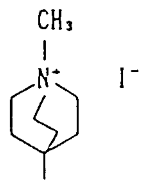 |
| 31 | 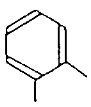 | – | 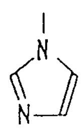 | – | 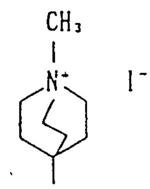 |
| 32 | 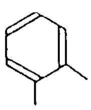 | – | 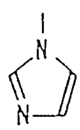 | – | 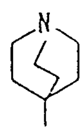 |
| 33 | 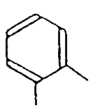 | – | 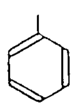 | – | 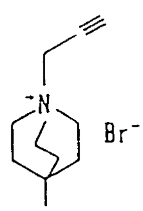 |

Formulation Example 1

| | |
|---|---|
| Compound of the present invention | 5.0 |
| Lactose | 113.6 |
| Microcrystalline cellulose | 28.4 |
| Light silicic anhydride | 1.5 |
| Magnesium stearate | 1.5 |

Using a DC type mixer, 15 g of the compound of the present invention was mixed with 340.8 g of lactose and 85.2 g of microcrystalline cellulose. The mixture was subjected to compression molding using a roller compactor to give a flake-like compressed material. The flake-like compressed material was pulverized using a hammer mill, and the pulverized material was screened through a 20 Mesh screen. A 4.5 g portion of light silicic anhydride and 4.5 g of magnesium stearate were added to the screened material and mixed using a DC type mixer. The mixed product was subjected to a tablet making machine equipped with a die/punch system of 7.5 mm in diameter, thereby obtaining 3,000 tablets, each having 150 mg in weight.

Formulation Example 2

| | |
|---|---|
| Compound of the present invention | 5.0 |
| Lactose | 95.2 |
| Corn starch | 40.8 |
| Polyvinylpyrrolidone K25 | 7.5 |
| Magnesium stearate | 1.5 |
| Hydroxypropylmethylcellulose 2910 | 2.3 |
| Polyethylene glycol 6000 | 0.4 |
| Titanium dioxide | 1.1 |
| Purified talc | 0.7 |

Using a fluidized bed granulating machine, 15 g of the compound of the present invention was mixed with 285.6 g of lactose and 122.4 g of corn starch. Separately, 22.5 g of polyvinylpyrrolidone was dissolved in 127.5 g of water to prepare a binding solution. Using a fluidized bed granulating machine, the binding solution was sprayed on the above mixture to give granules. A 4.5 g portion of magnesium stearate was added to the obtained granules and mixed using a DC type mixer. The obtained mixture was subjected to a tablet making machine equipped with a die/punch system of 7.5 mm in diameter, thereby obtaining 3,000 tablets, each having 150 mg in weight.

Separately, a coating solution was prepared by suspending 2.3 g of hydroxypropylmethylcellulose 2910, 0.4 g of polyethylene glycol 6000, 1.1 g of titanium dioxide and 0.7 g of purified talc in 24.2 g of water. Using a High Coater, the 3,000 tablets prepared above were coated with the coating solution to give film-coated tablets, each having 154.5 mg in weight.

Formulation Example 3

(Liquid inhalant)

A 10 mg portion of the compound of the present invention was dissolved in 90 ml of physiological saline, and the

solution was adjusted to a total volume of 100 ml with the same saline, dispensed in 1 ml portions into 1 ml capacity ampoules and then sterilized at 115°C for 30 minutes to give liquid inhalant.

Formulation Example 4

(Powder inhalant)

| | |
|---|---|
| Compound of the present invention | 50 µg |
| Lactose | 450 µg |
| Total | 500 µg |

A 5 g portion of the compound of the present invention was uniformly mixed with 45 g of lactose, and a 200 mg portion of the mixture was packed in a powder inhaler for exclusive use to produce a powder inhalant (500 µg per inhalation).

**Claims**

**1.** A carbamate derivative represented by the following general formula (I), a salt thereof, a hydrate thereof or a solvate thereof

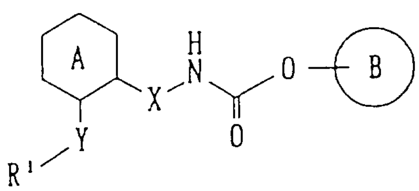

( I )

wherein each symbol has the following meaning:

A ring: a benzene ring or a pyridine ring,
B ring: a nitrogen-containing saturated hetero-ring which may have a substituent on the nitrogen atom and which may have a cross-linking,
$R^1$: a phenyl group which may have a substituent, a cycloalkyl or cycloalkenyl group having 3 to 8 carbon atoms, or a five- or six-membered nitrogen-containing heterocyclic group,
X: a single bond or a methylene group,
Y: a single bond, a carbonyl group, a methylene group which may be substituted with a hydroxyl group or a group represented by the formula $-S(O)_{\ell}-$, and
$\ell$: an integer of 0, 1 or 2.

**2.** The carbamate derivative or a salt thereof according to claim 1, wherein the B ring is a group represented by any one of the following general formulae (IIa), (IIb) and (IIc)

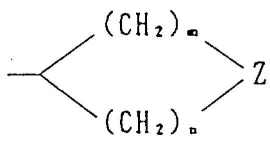

( II a )

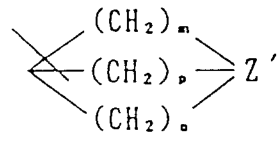

( II b )

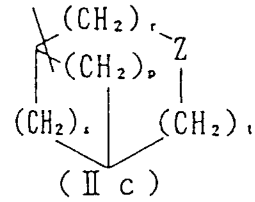

( II c )

wherein each symbol has the following meaning:

Z: a group represented by

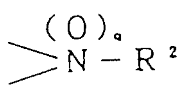

or

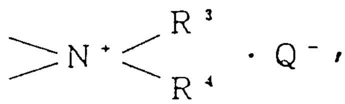

Z': a group represented by $\geqslant N(O)_q$ or $\geqslant N^+\text{-}R^5 \cdot Q^-$,
$Q^-$: an anion,
$R^2$: a hydrogen atom, a lower alkyl group, a lower alkenyl group, a lower alkynyl group, a cycloalkyl-lower alkyl group, an aralkyl group which may have a substituent, or a lower alkyl group substituted with a heterocyclic group which has 1 or 2 hetero atoms, which may have a substituent and which may be condensed,
$R^3$: a lower alkyl group, a lower alkenyl group, a lower alkynyl group, an aralkyl group which may have a substituent or a lower alkyl group substituted with a heterocyclic group which has 1 or 2 hetero atoms, which may have a substituent and which may be condensed,
$R^4$: a lower alkyl group, a lower alkenyl group or a lower alkynyl group,
$R^5$: a lower alkyl group, a lower alkenyl group, a lower alkynyl group or an aralkyl group,
m and n: these may be the same or different from each other and each is an integer of from 1 to 4 (with the proviso that m + n means an integer of from 3 to 5),
p: an integer of from 1 to 3 (with the proviso that m + p means an integer of from 3 to 5),
q: 0 or 1, and
r, s and t: these may be the same or different from one another and each is an integer of from 0 to 3 (with the proviso that r + s + t means 2 or 3).

3. The carbamate derivative or a salt thereof according to claim 2, wherein the B ring is a group represented by the general formula (IIb).

4. The carbamate derivative or a salt thereof according to claim 2, wherein the B ring is a group represented by the general formula (IIc).

5. The carbamate derivative or a salt thereof according to claim 1 or 2, wherein $R^1$ is a phenyl group which may have a substituent selected from a halogen atom, a nitro group, a cyano group, a trihalogenomethyl group, an amino group, a mono- or di-lower alkylamino group, a hydroxyl group, a mercapto group, a lower alkyl group, a lower alkenyl group, a lower alkynyl group and a lower alkoxy group or a five-membered nitrogen-containing heterocyclic group.

6. The carbamate derivative or a salt thereof according to claim 2, wherein the B ring is a group represented by the general formula (IIa),
and Z is a group represented by the formula

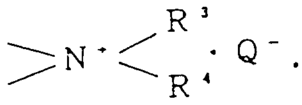

7. The carbamate derivative or a salt thereof according to claim 6, wherein $R^3$ and $R^4$ may be the same or different from each other and each represents a lower alkyl group.

8. A pharmaceutical composition which comprises the carbamate derivative of claim 1 or a pharmaceutically acceptable salt thereof as an active ingredient.

9. A muscarinic $M_3$ receptor antagonist, which comprises the carbamate derivative of claim 1 or a salt thereof as an active ingredient.

10. The muscarinic $M_3$ receptor antagonist according to claim 9, which is an agent for preventing or treating diseases in which the muscarinic $M_3$ receptor is concerned, including respiratory diseases such as chronic obstructive pulmonary disease, chronic bronchitis, asthma and rhinitis, urinary diseases such as urinary incontinence and pollakiuria in neurogenic pollakiuria, neurogenic bladder, nocturnal enuresis, unstable bladder, cystospasm and chronic cystitis and gastrointestinal diseases such as irritable bowel syndrome, spastic colitis and diverticulitis.

11. The muscarinic $M_3$ receptor antagonist according to claim 10, which is an agent for preventing or treating urinary diseases such as urinary incontinence and pollakiuria in neurogenic pollakiuria, neurogenic bladder, nocturnal enuresis, unstable bladder, cystospasm and chronic cystitis.

12. The muscarinic $M_3$ receptor antagonist according to claim 10, which is an agent for preventing or treating respiratory diseases such as chronic obstructive pulmonary disease, chronic bronchitis, asthma and rhinitis.

## INTERNATIONAL SEARCH REPORT

International application No.
PCT/JP95/00168

A. CLASSIFICATION OF SUBJECT MATTER Int. $Cl^6$ C07D211/46, 401/06, 405/06, 409/06, 451/04, 453/02, A61K31/435, 31/44, 31/445

According to International Patent Classification (IPC) or to both national classification and IPC

B. FIELDS SEARCHED

Minimum documentation searched (classification system followed by classification symbols) Int. $Cl^6$ C07D211/46, 401/06, 405/06, 409/06, 451/04, 453/02, A61K31/435, 31/44, 31/445

Documentation searched other than minimum documentation to the extent that such documents are included in the fields searched

Electronic data base consulted during the international search (name of data base and, where practicable, search terms used)

CAS ONLINE

C. DOCUMENTS CONSIDERED TO BE RELEVANT

| Category* | Citation of document, with indication, where appropriate, of the relevant passages | Relevant to claim No. |
|---|---|---|
| A | WO, A1, 93/20071 (Glaxo Group Ltd.), October 14, 1993 (14. 10. 93) & AU, A, 9339497 | 1-12 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| March 23, 1995 (23. 03. 95) | April 11, 1995 (11. 04. 95) |
| Name and mailing address of the ISA/ Japanese Patent Office | Authorized officer |
| Facsimile No. | Telephone No. |

Form PCT/ISA/210 (second sheet) (July 1992)